(19) Europäisches Patentamt
European Patent Office
Office européen des brevets

(11) Publication number: **0 182 608 B1**

(12) **EUROPEAN PATENT SPECIFICATION**

(45) Date of publication of patent specification: **03.03.93**

(51) Int. Cl.5: **C07D 333/74**, C07D 307/92, C07D 209/82, A61K 31/40, A61K 31/38, A61K 31/34

(21) Application number: **85308278.2**

(22) Date of filing: **14.11.85**

The file contains technical information submitted after the application was filed and not included in this specification

# (54) Polycyclic compounds, their preparation and formulations containing them.

(30) Priority: **15.11.84 GB 8428929**
**13.09.85 GB 8522754**

(43) Date of publication of application:
**28.05.86 Bulletin 86/22**

(45) Publication of the grant of the patent:
**03.03.93 Bulletin 93/09**

(84) Designated Contracting States:
**AT BE CH DE FR GB IT LI LU NL SE**

(56) References cited:
**EP-A- 0 125 701**
**EP-A- 0 125 702**
**DE-A- 2 510 001**
**US-A- 4 219 657**

**ARZNEIMITTELFORSCHUNG/DRUG RESEARCH, vol. 32(II), no. 9, 1982, pages 1013-1016; M. HRABOWSKA et al.: "Antitumor activity of 1-nitro-9-aminoacridine derivatives"**

(73) Proprietor: **THE WELLCOME FOUNDATION LIMITED**
**Unicorn House 160 Euston Road**
**London NW1 2BP(GB)**

(72) Inventor: **Bair, Kenneth Walter**
**342 Wesley Drive**
**Chapel Hill, North Carolina 27514(US)**

(74) Representative: **Rollins, Anthony John et al**
**Group Patents & Agreements The Wellcome Foundation Ltd Langley Court**
**Beckenham Kent BR3 3BS (GB)**

**EP 0 182 608 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to heteropolycyclic alkanol derivatives which have been found to have biocidal activity. More specifically the invention concerns aminoalkanol derivatives containing a heteropolycyclic ring system, methods for the synthesis thereof, novel intermediates thereof, pharmaceutical formulations thereof and the use thereof as biocidal agents, particularly antitumor agents.

Two analogues of nitracrine (1-nitro-9-((3-dimethylaminopropyl)amino)acridine) containing 2-amino-2-methyl-1,3-propanediol and tris(hydroxymethyl)methylamine groups have been reported to have antitumor activity in some murine screening systems (Arzneim. Forsch./Drug Res. 32II, 1013(1982)).

EP-A-125 701 and EP-A-125-702 describe carbocyclic polycyclic anti-tumour agents having an amino alkanol side chain.

We have now discovered a novel class of heteropolycyclic aromatic alkanol derivatives which have biocidal activity.

Accordingly, in a first aspect, the present invention provides a compound of the formula (I)

$ArCH_2NHR^1$ (I)

or a monomethyl or monoethyl ether thereof, the compound of formula (I) including the said ethers containing no more than 29 carbon atoms in total; an ester thereof; a salt thereof;
wherein Ar is a 6,6,6,5 tetracyclic aromatic ring system having 15 to 17 ring atoms, and containing one five membered ring in which there is one heteroatom, optionally substituted by one or two substituents, said substituents containing not more than four carbon atoms in total when taken together and being the same or different and are selected from halogen; cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(O)_nR^2$ wherein n is an integer 0,1 or 2 and $R^2$ is $C_{1-2}$ alkyl optionally substituted by hydroxy or $C_{1-2}$ alkoxy; or Ar is optionally substituted by a group $NR^3R^4$ containing not more than 5 carbon atoms wherein $R^3$ and $R^4$ are the same or different and each is a $C_{1-3}$ alkyl group or $NR^3R^4$ forms a five-or six-membered heterocyclic ring optionally containing one or two additional heteroatoms;
$R^1$ is

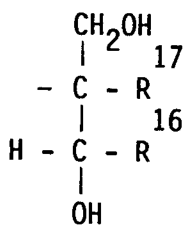

wherein $R^{16}$ is hydrogen or methyl and $R^{17}$ is hydrogen, methyl or ethyl, preferably methyl.

The heteroatom is preferably oxygen, sulphur or nitrogen. When it is nitrogen, this is substituted by hydrogen, methyl or ethyl; preferably by methyl or ethyl.

Aromatic ring systems within the scope of the present invention include:

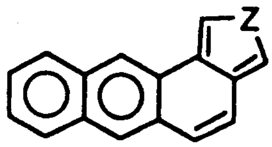

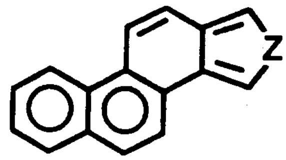

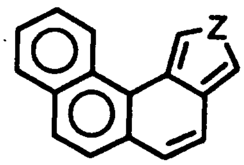

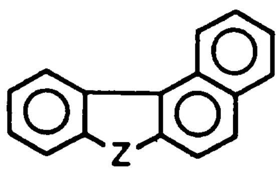

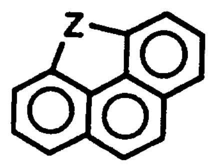

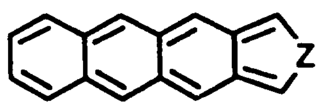

Z is a heteroatom

6,6,6,5-Tetracyclic Ring Systems

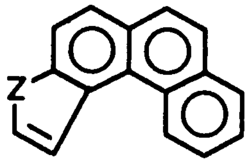

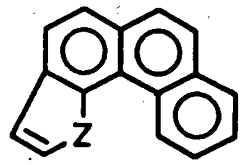

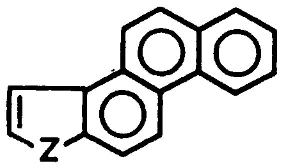

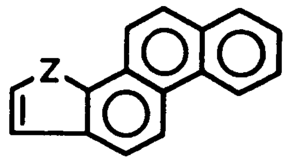

Z is a heteroatom

6,6,6,5-Tetracyclic Ring Systems

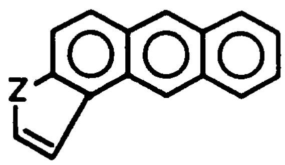

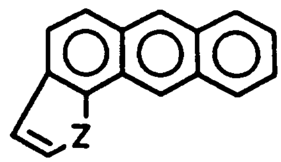

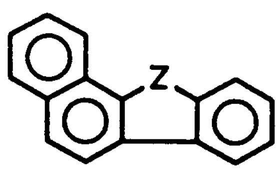

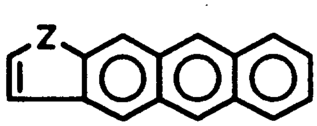

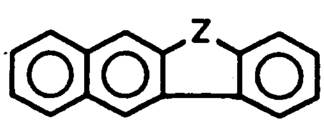

Z is a heteroatom

One preferred group of compounds of the present invention is that in which Ar is a ring system having 15 or 17 ring atoms.

One particularly preferred group of compounds of the formula (I) is that in which Ar is selected from:

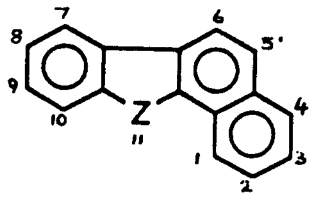

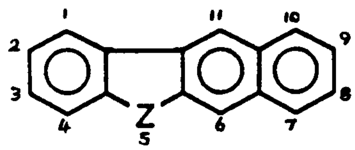

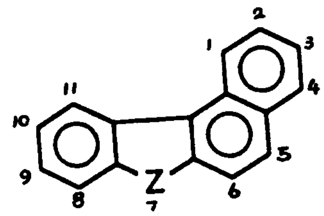

These ring systems have the following nomenclature:

| | | |
|---|---|---|
| | Z=NH | |
| 11-H-Benzo[a]carbazole | 5-H-Benzo[b]carbazole | 7-H-Benzo[c]carbazole |
| | Z=O | |
| Benzo[b]naphtho[2,1-d] furan | Benzo[b]naphtho[2,3-d] furan | Benzo[b]naphtho[1,2-d] furan |
| | Z=S | |
| Benzo[b]naphtho[2,1-d] thiophene | Benzo[b]naphtho[2,3-d] thiophene | Benzo[b]naphtho[1,2-d] thiophene |

Preferably the $CH_2NHR^1$ side chain is attached at one of the positions indicated below.

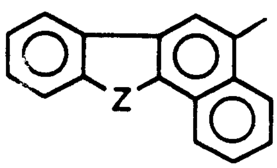

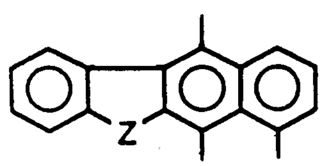

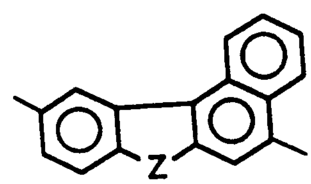

Suitably Ar is selected from

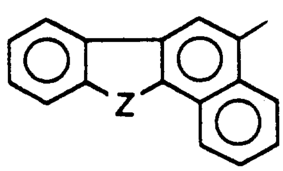

wherein Z = O, S, NMe or NEt

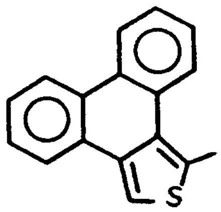

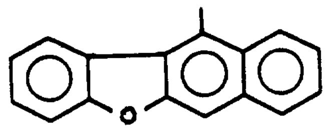

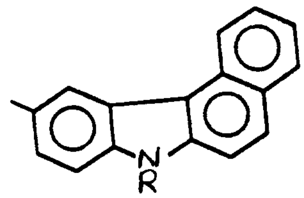

where R = Me or Et

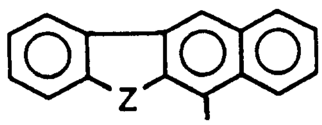

or

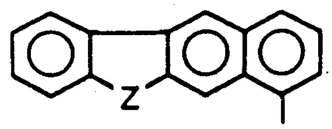

wherein Z = S or O and

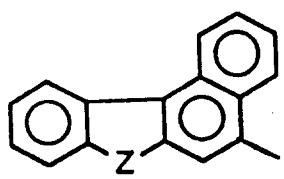

wherein Z = S,O, NMe, or NEt

A further preferred group of compounds of the formula (I) is that in which Ar is:

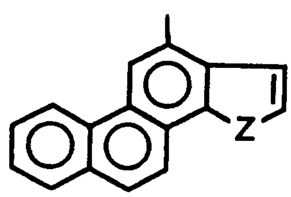

wherein Z = S,O,NMe or NEt

Preferably Ar is 7H-benzo[c]carbazol-10-yl or benzo[b]naphtho[2,1-d]furan-5-yl.

Suitably the aromatic ring is unsubstituted or has only one substituent. Preferably the aromatic ring is unsubstituted.

Suitably $ArCH_2NHR^1$ or a monomethyl or monoethyl ether thereof contains not more than 28 carbon atoms in total.

Specific compounds within the scope of formula (I) include;

2-[(9-Benzo[b]naphtho[2,1-d]thiophen-5-ylmethyl)amino]-2-methyl-1,3-propanediol
2-[(Benzo[b]naphthol[2,3-d]furan-6-ylmethyl)amino]-2-methyl-1,3-propanediol
2-[(Benzo[b]naphtho[1,2-d]furan-5-ylmethyl)amino]-2-methyl-1,3-propanediol
2-[(Benzo[b]naphtho[2,1-d]furan-5-ylmethyl)amino]-2-methyl-1,3-propanediol
2-Methyl-2-[((7-methyl-7-H-benzo[c]carbazol-10-yl)methyl)amino]-1,3-propanediol
2-((Benzo[b]naphtho[2,3-d]thiophen-6-ylmethyl)amino)-2-methyl-1,3-propanediol
2-(5-Ethyl-(benzo[b]carbazol-7-ylmethyl)amino)-2-methyl-1,3-propanediol
2-(Benzo[b]naphtho[2,3-d]furan-11-ylmethyl)amino)-2-methyl-1,3-propanediol
2-(Benzo[b]naphtho[2,3-d]thiophen-8-ylmethyl)amino)-2-methyl-1,3-propanediol

2-((5-Ethyl)-benzo[b]carbazol-6-ylmethyl)amino)-2-methyl-1,3-propanediol
2-((Benzo[b]naphtho[2,3-d]thiophen-7-ylmethyl)amino)-2-methyl-1,3-propanediol
2-((Phenanthro[9,10-c]thiophen-1-ylmethyl)amino)-2-methyl-1,3-propanediol
2-Methyl-2-[(phenanthro[9,10-b]furan-2-ylmethyl)amino]-1,3-propanediol
2-[(Benzo[b]naphtho[1,2-d]thiophen-5-ylmethyl)amino]-2-methyl-1,3-propanediol
2-Methyl-2-[(phenanthro[4,3-b]furan-2-ylmethyl)amino]-1,3-propanediol
2-[(Phenanthro[1,2-b]thiophen-2-ylmethyl)amino]-2-methyl-1,3-propanediol
2-Methyl-2-[(phenanthro[1,2-b]furan-2-ylmethyl)amino]-1,3-propanediol
2-Methyl-2-[(phenanthro[4,3-b]thiophen-7-ylmethyl)amino]-1,3-propanediol
2-[(Phenanthro[1,2-b]thiophen-4-ylmethyl)amino]-2-methyl-1,3propanediol
2-[(Phenanthro[1,2-b]furan-4-ylmethyl)amino]-2-methyl-1,3-propanediol
2-[Benzo[b]naphtho[2,3-d]furan-7-ylmethyl)amino]-2-methyl-1,3-propanediol
and monomethyl or monoethyl ethers, esters, and acid addition salts thereof.

Of these specific examples of compounds of formula (1) preferred compounds include 2-methyl-2-[7-methyl-7H-benzo[c]carbazol-10-yl)methyl]amino-1,3-propanediol and 2-((benzo[b]naphtho[2,1-d]furan-5-ylmethyl)amino)-2-methyl-1,3-propanediol and monomethyl or monoethyl ethers, esters and acid addition salts thereof.

Salts included within the scope of the present invention are those of compounds of formula (1) and ethers and esters thereof.

Esters and non-pharmaceutically useful salts of the compounds of the formula (I) are useful intermediates in the preparation and purification of compounds of the formula (I) and pharmaceutically useful salts thereof, and are therefore within the scope of the present invention. Thus, salts of the compounds of the formula (I) useful in the present invention include but are not limited to those derived from inorganic acids, such as hydrochloric, hydrobromic, sulfuric and phosphoric acids, and organic acids such as isethionic (2-hydroxyethylsulfonic), maleic, malonic, succinic, salicylic, tartaric, lactic, citric, formic, lactobionic, pantothenic, methanesulfonic, ethanesulfonic, benzenesulfonic, p-toluenesulfonic, naphthalene-2-sulfonic, and ascorbic acids, and amino acids such as glycine. Pharmacologically and pharmaceutically acceptable salts are preferred, particularly those that are soluble in solvents suitable for parenteral administration, for example, hydrochlorides, methanesulfonates and isethionates.

Esters of compounds of formula (I) are derived from acids known to those skilled in the art to be suitable for ester formation, and are conveniently those derived from $C_{1-6}$ alkanoic acids or alkanoic acid derivatives, for example acetic acid, propionic acid, n-butyric acid and iso-butyric acid. The esters may be formed from all or only some of the hydroxy groups contained in the compounds of formula (I).

The compounds of formula (I) and their ethers, esters, and salts thereof may be prepared by any method known in the art for the preparation of compounds of analogous structure. Thus, the compounds of formula (I) may, for example, be prepared by any of the methods defined below.

1. The reduction of a compound

$ArCH=NR^1$ (II)

wherein Ar and $R^1$ are as hereinbefore defined, or an appropriately protected derivative thereof followed by deprotection where appropriate.

The conditions and reagents for such a reaction are well known to those skilled in the art, and any such conditions/reagents may be employed that will not reduce the aromatic ring system. The conversion of (II) or suitably protected derivatives thereof may be carried out by a reducing agent followed by deprotection if necessary. The reduction is conveniently carried out by a metal hydride such as lithium aluminum hydride, sodium borohydride, sodium cyanoborohydride, or by catalytic hydrogenation, conveniently by hydrogen in the presence of a metal catalyst such as palladium or platinum, or equivalent reagents as outlined by J. March, Advanced Organic Chemistry, 2nd ed., pages 819-820, McGraw Hill, New York, 1977. The reduction is suitably carried out with $Ar-CH=NR^1$ in solution in an inert solvent or mixture of solvents compatible with the reducing agent, at a non-extreme temperature, for example, between 0° and 80°C, conveniently at room temperature.

In the case of lithium aluminum hydride and like reagents, suitable solvents include ethers (for example tetrahydrofuran, diethyl ether and 1,2-dimethoxyethane) optionally in the presence of a hydrocarbon cosolvent (for example toluene, benzene or hexane).
In the case of sodium borohydride and like reagents, suitable solvents include alcohols (for example ethanol, methanol or isopropanol) optionally in the presence of a hydrocarbon cosolvent (for example toluene, benzene or hexane) or an ether cosolvent (for example diethylether or tetrahydrofuran).

In the case of sodium cyanoborohydride and like reagents, suitable solvents include those described for sodium borohydride and the reduction is conveniently carried out in the presence of an acid, conveniently glacial acetic acid or ethanolic hydrochloric acid as outlined in, for example, R. Hutchins et al., Organic Preparations and Procedures International 11, 201(1979).

In the case of catalytic hydrogenation, suitable solvents include alcohols (for example methanol and ethanol) optionally in the presence of a hydrocarbon cosolvent (for example toluene or benzene), or ether cosolvent (for example diethyl ether or tetrahydrofuran) optionally in the presence of an acid (for example glacial acetic acid or ethanolic hydrochloric acid), or glacial acetic acid.

Protected derivatives of compounds $ArCH=NR^1$ are conveniently used when lithium aluminum hydride is employed as the reducing agent. Convenient protecting groups are compatible with the reducing agent utilized and are readily removed under nondestructive conditions, for example benzyl, tetrahydropyranyl and isopropylidene ethers.

It is often convenient not to isolate the compound $ArCH=NR^1$ but to react a compound ArCHO with a compound $NH_2R^1$; wherein Ar and $R^1$ are as defined in (1) and to reduce the compound of the formula $ArCH=NR^1$ so Formed in situ. The reaction of the compounds ArCHO and $NH_2R^1$ is again suitably carried out using conditions and reagents which are well known to those skilled in the art, for example in the presence of an acid, such as a sulfonic acid, i.e. p-toluenesulfonic acid, in an appropriate inert solvent, such as an aromatic hydrocarbon, suitably toluene, with azeotropic removal of water followed by treatment with the reducing agent in an appropriate solvent, suitably ethanol or methanol. Alternatively, $ArCH=NR^1$ formed under equilibrium conditions in appropriate solvents can be reduced in situ with an appropriate reducing agent, suitably sodium cyanoborohydride.

The compound ArCHO may be in the form of a protected aldehyde, for example an acetal, which liberates the aldehyde function under the reaction conditions. In turn, a compound ArCHO can be synthesized by reacting the appropriate polycyclic aromatic hydrocarbon with a formylating agent such as that generated by the reaction between $SnCl_4$ and $Cl_2CHOCH_3$ or equivalent reagents, for example, according to the method of A. Reiche et al., Chem. Ber. 93, 88(1960), or with other standard formylating reagents/procedures known to the art, for example, the Gatterman-Koch reaction ($CO/HCl/AlCl_3/CuCl$), the Gatterman reaction ($HCN/HCl/ZnCl_2$), and the Vilsmeier reaction ($POCl_3/PhN(Me)CHO$ or $POCl_3/Me_2NCHO$)(J.March, vide supra pages 494-497).

The compounds ArCHO may also be prepared from an appropriate aromatic heteropolycycle substituted by a suitable functional group and converting this functional group to an aldehyde group by methods well known to those skilled in the art. Suitable functional groups include $CHBr_2$, $CH_3$, $COR^{19}$ wherein $R^{19}$ is a primary or secondary $C_{1-6}$ alkyl group, COOH or a derivative thereof such as an ester, amide, or acid chloride, or CN.

Where the aromatic heteropolycycle bears substituents, ArCHO may be prepared by a variety of methods known in the art of organic chemistry depending on the nature of the substituent on the ring. For example, if the substituent(s) is a halogen, the starting materials may be prepared by direct treatment of the aromatic heteropolycycle with a halogenating agent (e.g. $Cl_2$, $Br_2$, or $SO_2Cl_2$) or indirectly by such routes as the Sandmeyer reaction (H.H. Hodgson, Chem. Rev. 40, 251 (1947). If the substituent(s) is alkyl, the aromatic heteropolycycle may be reacted with the appropriate reagents under Friedel-Crafts reaction conditions (G.A. Olah, Friedel Crafts and Related Reactions, Vols. 1-3, Interscience, New York, NY, 1963-1965).

The compounds of the formula $NH_2R^1$ also may be prepared by methods known in the art, for example, when $R^1$ is as hereinbefore defined, by the reaction of $NO_2R^{18}$, wherein $R^{18}$ is

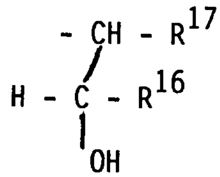

wherein $R^{16}$ and $R^{17}$ are as hereinbefore defined, with an appropriate aldehyde, conveniently acetaldehyde or formaldehyde (as in B.M. Vanderbilt and H.B. Hass, Ind. Eng. Chem. 32, 34(1940)) followed by reduction (as outlined in J. March, vide supra, pages 1125-1126), conveniently by hydrogen and a metal catalyst (for example, a platinum containing catalyst) in an appropriate solvent, conveniently glacial acetic acid.

2. The reduction of a compound Ar. CO. $NHR^1$
wherein Ar and $R^1$ are as hereinbefore defined and the hydroxy groups are optionally protected, followed by deprotection of the hydroxy groups where appropriate. The reduction may be carried out by standard reducing agents known for carrying out this type of reduction that will not reduce the aromatic ring system (as outlined in J. March, vide supra, page 1122), for example, a hydride reagent such as lithium aluminium hydride in an inert solvent, such as an ether, i.e. tetrahydrofuran, at a non-extreme temperature, for example, at between 0° and 100°C and conveniently at the reflux temperature of the ether.

The compound Ar. CO. $NHR^1$ may be formed by the reaction of the appropriate acid (ArCOOH) or a suitable reactive acid derivative thereof as outlined in J. March, vide supra, pages 382-390) for example, an acid halide in an inert solvent, with an amine $NH_2R^1$ in which the hydroxy groups are optionally protected, for example, when the compound $NH_2R^1$ is a diol, by an isopropylidene group. The compound Ar. CO. $NHR^1$ so formed is suitably reduced in situ and deprotected if necessary to give a compound of formula (I). The compounds of the formula ArCOOH can be prepared by methods well known to those skilled in the art.

3. The reaction of a compound $ArCH_2L$ (wherein Ar is as hereinbefore defined and L is a leaving group), with a compound $NH_2R^1$ as hereinbefore defined. Suitable leaving groups are those defined by J. March, vide supra pages 325-331, and include halogens such as chlorine or bromine and sulfonic acid derivatives such as p-toluenesulfonate. The reaction is suitably carried out in an appropriate solvent, such as a dipolar aprotic solvent or alcohol at a non-extreme temperature, for example 50-100°. The compounds of the formula $ArCH_2L$ can be prepared by methods well known to those skilled in the art.

There is therefore provided, as a Further aspect of the invention, a method for the preparation of a compound of formula (I) comprising any method known for the preparation of analogous compounds, in particular those methods defined in (1) to (3) hereinabove.

In a further aspect, the present invention provides novel chemical intermediates $ArCH=NR^1$, Ar. CO. $NHR^1$ or $ArCH_2L$ as herein before defined. Compounds of the formula (I) in which one or more of the hydoxy groups are protected, for example by benzyl, trityl or isopropylidene groups are also useful intermediates of the present invention.

The compounds of this invention have biocidal activity, e.g. are toxic to certain living cells which are detrimental to mammals, for example pathogenic organisms and tumours.

This toxicity to pathogenic organisms has been demonstrated for example, by activity against one or more of the following: viruses (e.g. Herpes simplex 1/vero), fungi (e.g. Candida albicans), protozoa (e.g. Eimeria tenella and Trichomonas vaginalis), bacteria (e.g. Mycoplasma smeg matis and Streptococcus pyogenes), and helminths (e.g. Nippostrongylus brasiliensis and Brugia pahangi). The antitumour activity of compounds of formula I has been demonstrated in a number of recognized screens and primarily by activity against ascitic P388/O leukaemia.

Preferred compounds of the formula (I) are those which have antitumour activity. The activity against ascitic tumours, including P388/O, is evidenced by reduction of tumour cell number in mammals (for example, mice bearing ascitic tumours) and their consequent increase in survival duration as compared to an untreated tumour bearing control group. Antitumour activity is further evidenced by measurable reduction in the size of solid tumours following treatment of mammals with the compounds of this invention compared to the tumours of untreated control tumour bearing animals. Compounds of formula (I) are active against murine tumours such as lymphocytic leukaemia P388/0, lymphocytic leukaemia L1210, melanotic melanoma B16, P815 mastocytoma, MDAY/D2 fibrosarcoma, colon 38 adenocarcinoma, M5076 rhabdomyosarcoma and Lewis lung carcinoma.

Activity in one or more of these tumour tests has been reported to be indicative of antitumour activity in man (A. Goldin et al. in Methods in Cancer Research ed. V.T. DeVita Jr. and H. Busch, 16, 165, Academic Press, N.Y. 1979).

There are sublines of P388/0 which have been made resistant to the following clinically useful agents: cytosine arabinoside, doxorubicin, cyclophosphamide, L-phenylalanine mustard, methotrexate, 5-fluorouracil, actinomycin D, cis-platin and bis-chloroethylnitrosourea. Compounds of this invention show potent activity against these drug-resistant tumours using the procedure for P388/O above.

Compounds of formula (I) have also been found to be active against human tumour cells in primary cultures of lung, ovary, breast, renal, melanoma, unknown primary, gastric, pancreatic, mesothelioma, myeloma, and/or colon cancer. (As used herein "cancer" is to be taken as synonymous with "malignant tumour" or more generally "tumour" unless otherwise noted.) This is a procedure in which the prevention of tumour cell colony formation, i.e. tumour cell replication, by a drug has been shown to correlate with clinical antitumour activity in man (D.D. Von Hoff et al., Cancer Chemotherapy and Pharmacology 6, 265 (1980); S.

Salmon and D.D. Von Hoff, Seminars in Oncology, 8, 377 (1981)).

Compounds of formula I which have been found to have antitumour activity intercalate in vitro with DNA (this property is determined by viscometric methods using the procedure of W. D. Wilson et al., Nucleic Acids Research 4, 2697 (1954)) and have a log P as calculated by the method of C. Hansch and A.Leo in Substituent Constants for Correlation Analysis in Chemistry and Biology, John Wiley and Sons, New York, 1979, lying in the range between -2.0 and +2.5.

As has been described above, the compounds of the present invention are useful for the treatment of tumours. The invention thus further provides a method for the treatment of tumours in animals, including mammals, especially humans, which comprises the administration of a clinically useful amount of compound of formula (I) in a pharmaceutically useful form, once or several times a day or other appropriate schedule, orally, rectally, parenterally, or applied topically. In addition, there is provided as a further, or alternative, aspect of the invention, a compound of formula (I) for use in therapy, for example as an antitumour agent.

The amount of compound of formula (I) required to be effective as a biocidal agent will, of course, vary and is ultimately at the discretion of the medical or veterinary practitioner. The factors to be considered include the condition being treated, the route of administration, and nature of the formulation, the mammal's body weight, surface area, age and general condition, and the particular compound to be administered. A suitable effective antitumour dose is in the range of about 0.1 to about 120 mg/kg body weight, preferably in the range of about 1.5 to 50 mg/kg, for example 10 to 30 mg/kg. The total daily dose may be given as a single dose, multiple doses, e.g., two to six times per day or by intravenous infusion For selected duration. For example, for a 75 kg mammal, the dose range would be about 8 to 9000 mg per day, and a typical dose would be about 2000 mg per day. If discrete multiple doses are indicated, treatment might typically be 500 mg of a compound of formula I given 4 times per day in a pharmaceutically useful formulation.

Whilst it is possible for the active compound (defined herein as compound of formula (I), or ether, ester, or salt thereof) to be administered alone, it is preferable to present the active compound in a pharmaceutical formulation. Formulations of the present invention, for medical use, comprise an active compound together with one or more pharmaceutically acceptable carriers thereof and optionally other therapeutical ingredients. The carrier(s) must be pharmaceutically acceptable in the sense of being compatible with the other ingredients of the formulation and not deleterious to the recipient thereof.

The present invention, therefore, further provides a pharmaceutical formulation comprising a compound of formula (I) (in the form of the free base, ether, or ester derivative or a pharmaceutically acceptable acid addition salt thereof) together with a pharmaceutically acceptable carrier therefor.

There is also provided a method for the preparation of a pharmaceutical formulation comprising bringing into association a compound of formula (I) an ether, ester, or pharmaceutically acceptable salt thereof, and a pharmaceutically acceptable carrier therefor.

Whilst the antitumour activity of the compounds of Formula (I) is believed to reside in the free base, it is often convenient to administer an acid addition salt of a compound of formula (I).

The formulations include those suitable for oral, rectal or parenteral (including subcutaneous, intramuscular and intravenous) administration. Preferred formulations are those suitable for oral or parenteral administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into assocation with a liquid carrier or a finely divided solid carrier or both and then, if necessary, shaping the product into desired formulations.

Formulations of the present invention suitable for oral administration may be presented as discrete units such as capsules, cachets, tablets or lozenges, each containing a predetermined amount of the active compound; as a powder or granules; or a suspension in an aqueous liquid or non-aqueous liquid such as a syrup, an elixir, an emulsion or a draught.

A tablet may be made by compression or moulding, optionally with one or more accessory ingredients. Compressed tablets may be prepared by compressing in a suitable machine the active compound in a free-flowing form such as a powder or granules, optionally mixed with a binder, lubricant, inert diluent, surface active or dispersing agent. Moulded tablets may be made by moulding in a suitable machine, a mixture of the powdered active compound with any suitable carrier.

A syrup may be made by adding the active compound to a concentrated, aqueous solution of a sugar, for example sucrose, to which may also be added any accessory ingredients. Such accessory ingredient(s) may include flavourings, an agent to retard crystallization of the sugar or an agent to increase the solubility

of any other ingredient, such as a polyhydric alcohol for example glycerol or sorbitol.

Formulations for rectal administration may be presented as a suppository with a conventional carrier such as cocoa butter.

Formulations suitable For parenteral administration conveniently comprise a sterile aqueous preparation of the active compound which is preferably isotonic with the blood of the recipient. Such formulations suitably comprise a solution of a pharmaceutically and pharmacologically acceptable acid addition salt of a compound of the Formula (I) that is isotonic with the blood of the recipient. Thus, such formulations may conveniently contain distilled water, 5% dextrose in distilled water or saline and a pharmaceutically and pharmacologically acceptable acid addition salt of a compound of the formula (I) that has an appropriate solubility in these solvents, For example the hydrochloride, isethionate and methanesulfonate salts, preferably the latter.

Useful formulations also comprise concentrated solutions or solids containing the compound of formula (I) which upon dilution with an appropriate solvent give a solution suitable for parenteral administration as above.

In addition to the aforementioned ingredients, the formulations of this invention may Further include one or more accessory ingredient(s) selected from diluents, buffers, flavouring agents, binders, surface active agents, thickeners, lubricants, preservatives (including antioxidants) and the like.

The following examples are provided by the way of illustration of the present invention and should in no way be construed as a limitation thereof.

## General Comments

All solvents were reagent grade and used without further purification with the following exceptions. Tetrahydrofuran (THF) was dried by distillation from Na/K alloy under nitrogen ($N_2$) and used immediately. Toluene ($PhCH_3$) was distilled from $CaH_2$ under $N_2$ and stored over 3A molecular sieves. Chemicals used were reagent grade and used without purification unless noted. The full name and address of the suppliers of the reagents and chemicals is given when first cited. After this, an abbreviated name is used.

Preparative HPLC was carried out on a Waters Prep LC/System 500A machine using two 500 g silica gel ($SiO_2$) cartridges unless otherwise noted. Plugs of $SiO_2$ used for purifications were "flash chromatography" silica gel (Merck Co., Inc., Merck Chemical Division, Rahway, NJ, 07065 silica gel 60, 230-400 mesh). An appropriate volume sintered glass funnel was filled approximately 3/4 full with the silica gel and packed evenly by tapping the outside of the funnel. A piece of filter paper was then placed on top of the silica gel and a solution of the material to be purified applied evenly to the top. Gentle suction through a filter flask moved the eluting solvent through the plug rapidly. The appropriate size fractions were combined as needed and further manipulated.

General procedures are described in detail. Analogous procedures show melting point (mp), recrystallization solvents, and elemental analyses (all elements analyzing within a difference of ±0.4% of the expected value). Any changes to the procedure such as solvent, reaction temperature, reaction time, or workup are noted.

NMR ($^1H$, $^{13}C$), IR, MS data of all new products were consistent with the expected and proposed structures. The positions assigned to structural isomers were unequivocally determined by a number of NMR techniques. All final products were dried in a vacuum oven at 26.7 mbar (20 mm Hg) pressure at the temperature indicated overnight (12-16 hours). All temperatures are in degrees Celsius.

## EXAMPLE 1

### 2-((Benzo[b]naphtho [2,1-d] thiophen-5-ylmethyl)amino)-2-methyl-1,3-propanediol methanesulfonate

To a round bottomed flask equipped with magnetic stirring bar, condenser, thermometer, Dean-Stark trap, and nitrogen inlet and bubbler was added benzo[b]naphtho[2,1-d)thiophene-5-carbaldehyde, (SISA Pharmaceutical Laboratories, Inc; 763 Concord Avenue, Cambridge, MA, 02138, 4.94g, 18.83 mmol), 2-amino-2-methyl-1,3-propanediol (Aldrich, 1.98g, 18.83 mmol), p-toluenesulfonic acid monohydrate (Aldrich, 0.1g) and toluene (200 mL). The mixture was stirred at reflux with removal of water for 2.5h (or until no water is formed which was extracted with ethyl acetate (3x500 mL). The ethyl acetate collected). Most of the toluene was then removed by distillation. The mixture was then cooled in an ice bath and diluted with abs. ethanol (200 mL) and further cooled. Solid sodium borohydride (MCB Manufacturing Chemists, Inc., 2909 Highland Ave., Cincinnati, OH, 45212, 0.712g, 18.83 mmol) was added in one portion to the reaction mixture. The ice bath was then removed, the reaction mixture allowed to warm to room temperature and

then stirred overnight. The reaction was then acidified with 10% hydrochloric acid and the solvents removed by rotary evaporation. The crude solid was shaken with IN hydrochloric acid (300 mL) filtered, washed with hydrochloric acid, sucked semidry and washed with diethyl ether (300 mL). The material was dissolved in methanol (200 mL), filtered and basified with 1N sodium hydroxide solution (1L). A white solid washings were combined, Filtered, washed with satd. sodium chloride (3x500 mL), dried with potassium carbonate (Mallinkrodt, 25g), filtered, and concentrated by rotary evaporation to give a white solid. This was dissolved in a mixture of abs. ethanol (200 mL) and methanesulphonic acid (99.5%, Morton Thiokol, Inc. - Alfa Products, PO Box 299, 152 Andover Street, Danvers, MA, 01923, 3 mL), filtered and diluted to 4L with a mixture of diethyl ether/hexane (1:1). This material was recrystallized three times from ethanol/hexane (1:3) to give 2-(benzo[b]naphtho[2,1-d]thiophen-5-ylmethyl)amino-2-methyl-1,3-propanediol methanesulfonate mp 221-222° which analysed correctly for the assigned structure (C,H,N,S).

EXAMPLE 2

2-((Benzo[b]naphthol[2,3-d]furan-6-ylmethyl)amino)-2-methyl-1,3-propanediol

2A Benzo[b]naphtho[2,3-d]furan-6-carbaldehyde

Benzo[b]naphtho[2,3-d]furan (SISA) was formylated using the procedure of A. Rieche et al, Chem. Ber. 93, 88(1960). The crude aldehyde appeared to be mainly one isomer by TLC. Purification by chromatography, silica gel, toluene followed by recrystallization (methylene dichloride/hexane) gave pure material (58% yield) identified using NMR techniques to be benzo[b]naphtho[2,3-d]furan-6-carbaldehyde mp 169-171.5°. The product analysed correctly for the assigned structure (C,H).

2B. 2-((Benzo[b]naphtho[2,3-d]furan-6-ylmethyl)amino]2-methyl-1,3-propanediol methanesulfonate

Using the reductive amination procedure described in exmple 1, benzo[b]naphtho[2,3-d]furan-6-carbaldehyde (2A) and 2-amino-2-methyl-1,3-propanediol (Aldrich) gave 2-((benzo[b]naphtho[2,3-d]furan-6-ylmethyl)amino)-2-methyl-1,3-propanediol methanesulfonate 0.4 $H_2O$ mp 187-190°, (EtOH/$Et_2O$) which analysed correctly for the assigned structure (C,H,N,S).

EXAMPLE 3

2-((Benzo[b]naphtho[1,2-d]furan-5-ylmethyl)amino-2-methyl-1,3-propanediol

3A Benzo[b]naphtho[1,2-d]furan-5-carbaldehyde.

Benzo[b]naphtho[1,2-d]furan (SISA) was formylated using the procedure of A. Rieche et al, Chem. Ber. 93, 88(1960). The cruse aldehyde appeared o be only one isomer by TLC. Purification by chromatography (silica, toluene) followed by recrystallization (methylene dichloride/hexane) gave pure material (39% yield), indentified using NMR techniques to be benzo[b]-naphtho[1,2-d]furan-5-carbaldehyde mp 143-145°. The product analysed correctly for the assigned structure (C, H).

3B 2-((Benzo[b]naphtho[1,2-d]furan-5-ylmethyl)amino)-2-methyl-1,3-propanediol methanesulfonate

Using the reductive amination procedure described in example 1, benzo[b]naphtho[1,2-d]furan-5-carbaldehyde (3A) and 2-amino-2-methyl-1,3-propanediol (Aldrich) gave 2-((benzo[b]naphtho[1,2-d]furan-5-ylmethyl)amino)-2-methyl-1,3-propanediol methanesulfonate mp 215-217°, (EtOH/$Et_2O$) which analysed correctly for the assigned structure (C,H,N,S).

Example 4

2-Methyl-2-[[(7-methyl-7H-benzo[c]-carbazol-10-yl)methyl]amino-1,3-propanediol

4A 7-Methyl-7H-benzo[c]carbazole

7H-Benzo[c]carbazole (6.6g) (ex. PARS) was dissolved in tetrahydrofuran (250mL) under nitrogen and treated with potassium t-butoxide (4.2g). Once this had dissolved, dimethylsulphate (7.56g) was added and the mixture stirred for several minutes after which time TLC (silica/toluene) showed the reaction to be complete. The reaction mixture was poured into 1N sodium hydroxide (2L) and stirred. The solid so formed was collected by filtration, taken up in toluene (3000mL) and eluted through a plug of silica (2"x2") with toluene. The product containing fractions were evaporated to dryness, the solid dissolved in methylene chloride (400mL) and diluted with hexane. Concentration under vacuum gave a white solid (5.0g) which was filtered and washed with pentane. A further 1.06g of product (total yield 6.06g, 87%) was obtained on standing and further concentration of the filtrate. Analysis $C_{17}H_{13}N$ calculated: C,88.28;

H, 5.67, N; 6.06%, found: C, 88.31; H 5.68; N 6.01%.

4B 7-Methyl-7-H-benzo[c]carbazole-10-carbaldehyde

7-Methyl-7H-benzo-[c]carbazole (5.8g) was dissolved in methylene chloride and treated with stannic chloride (13.0g) under nitrogen. The mixture was cooled and α,α-dichloromethyl methyl ether (3.74g) was added dropwise. The reaction was stirred at RT for 4 hours, then heated to reflux for 15 minutes. TLC (silica/toluene) showed complete reation; the reaction mixture was cooled, water (300mL) added and the mixture stirred for 2 hours. The 2-phase mixture was filtered and the organic layer isolated and evaporated to dryness. The residue was dissolved in toluene (100mL) and eluted through a plug of silica (5"x3") with toluene. The ninth litre eluted contained the major isomer present and this and the tenth litre (eluted with dichloromethane) were combined and evaporated to dryness. The residue was taken up in dichloromethane (200mL), filtered and diluted 1:1 with pentane. The solution was evaporated until crystallisation began. Further pentane was added and the volume reduced to 150mL. Filtration afforded the title product (3.83g) as a pale yellow solid, m.p. 164-165°, pure by t.l.c. Analysis $C_{18}H_{13}NO$ calculated: C, 83:37; H,5.05; N, 5.40%; found: C,83.19; H,5.07; N,5.36%.

The 3rd through 8th litres eluted from the silica plug above were combined and evaporated to dryness. The solid was taken up in dichloromethane (200mL), then diluted with hexane (200mL). Evaporation of the solvent gave a yellow solid which was dissolved in dichloromethane (200mL), diluted with hexane (200mL) and concentrated to give a crystalline yellow solid (0.55g) which was identified by NMR as 7-methyl-7H-benzo[c]carbazole-5-carbaldehyde, m.p.210-213°. Analysis $C_{18}H_{13}NO$ calculated: C, 83:37; H,5.05; N, 5.40%; found: C,83.43; H,5.08; N,5.37%.

4C 2-Methyl-2-[[(7-methyl-7-H-benzo[c]carbazol-10-yl) methyl]amino-1,3-propanediol hydrochloride 3/10 hydrate.

The title compound (4) was prepared by a method analogous to Example 1 from 7-methyl-7H-benzo[c]-carbazole-10-carbaldehyde and 2-amino-2-methyl-1,3-propanediol, m.p. 229-230° (dec)(MeOH:$Et_20$/1:3). Analysis $C_{22}H_{15}N_20_2Cl.3/10H_20$calc ulated; C,67.70; H,6.61; N,7.18; Cl, 9.08%; found: C, 67.61; H.6.53; N,7.17; Cl, 9.05%. NMR spectrum was consistent with this structure.

## Example 5

## 2-[(Benzo[b]naphtho[2,1-d]furan-5-ylmethyl)amino-2-methyl)-1,3-propanediol

5A Benzo [b]naphtho[2,1-d]furan-5-carboxaldehyde

Benzo[b]naphtho[2,1-d]furan (6.15g, PARS) was dissolved in methylene chloride (400mL) under nitrogen. Stannic chloride (14.59g) was added followed by α,α-dichloromethyl methyl ether (4.02g), and the resulting mixture stirred overnight and then refluxed for 3 minutes. Water (200mL) was added and the mixture stirred overnight. The organic layer was separated and evaporated to dryness. The residue was dissolved in toluene (200mL) and eluted through a plug of silica (3 1/2" x 3") with toluene. The product fractions were collected, evaporated to dryness, redissolved in methylene chloride (200mL), diluted with pentane (500mL) and concentrated to 150mL. Additional pentane (200mL) was added to the precipitate which was then filtered to give the title compound (5.0g), m.p. 118-121°. A further 0.34g of product was isolated from the filtrate. Analysis $C_{17}H_{10}O_2$ calculated: C,82.91; H,4.09%, Found: C,83.00; H,4.13%. NMR spectrum consistent with structure.

5B 2-[(Benzo[b]naphtho[2,1-d]furan-5-ylmethyl)amino-2-methyl-1,3-propanediol The title compound was prepared as its methanesulphonate salt(s) by a method analogous to example 1 from benzo[b]naphtho-[2,1-d]furan-5-carboxaldehyde and 2-methyl-1,3propanediol, m.p. 216-218° (EtOH:$Et_20$/1:4). Analysis $C_{22}H_{25}NO_6S$ calculated: C,60.23; H, 5.93; N, 3.19; S, 7.31%; found: C, 60.22; H, 5.93; N, 3.18; S,7.30% NMR spectrum was consistent with structure.

## Example 6

## 2-[(Benzo[blnaphtho[2,3-d]furan-7-ylmethyl)amino[-2-methyl-1,3-propanediol

6A 7-Bromomethyl-benzo[b]naphthol[2,3-d]furan

To a round bottom flask was added 7-methyl-benzo[b]naphtho-[2,3-d]furan (16.0g, 0.07 mol, ex. Pars), N-bromosuccinimide (12.8g, 0.072 mol), a catalytic amount of benzoyl peroxide (0.01g) and carbon tetrachloride (1L). The mixture was refluxed for 2.5 hours, cooled and filtered to remove the succinimide formed in the reaction. The solvent was then removed from the reaction mixture by rotary evaporation. The crude product was purfied by flash chromatography on silica gel using toluene as the eluting

solvent. The appropriate fractions were combined and the solvent once again removed by rotary evaporation to give 22.0g of product. The material (which was one spot by thin layer chromatography and pure by NMR) was used without further purification.

6B 2-[(Benzo[b]naphthol[2,3-d]furan-7-ylmethyl)amino]-2-methyl-1,3-propanediol methanesulphonate 1/4 hydrate

To a round bottom flask was added 7-bromomethyl-benzo[b]naththol[2,3-d]furan (22.0g, 0.0706 mol), potassium carbonate (19.49g, 0.141 mol) and absolute ethanol (600 mL). The mixture was refluxed overnight, cooled and filtered. The solvent was then removed by rotary evaporation to give a white residue. This was shaken with hot water (500 mL). The mixture was allowed to stand at room temperature for 1 hour and the resulting solid filtered. The mixture was filtered and the resulting solid washed twice with warm water (500 mL.) The damp solid was dissolved in absolute ethanol (400 mL) containing methanesulphonic acid (3 mL). The liquid was filtered through a fine fritted glass funnel and the filtrate diluted to 2L with ether. The resulting solid was filtered and recrystallised twice from absolute ethanol/ether (1/2). After drying in a vacuum oven overnight at 80°, 9.67g of the title compound (6) (32%) m.p. 248-249° (dec) was obtained. Analysis $C_{22}H_{25}NO_6S$ 1/4$H_20$ calculated: C,60.48; H,5.95; N,3.17; S,7.30%, found; C,60.60; H,5.89; N,3.21; S,7.35%.

## Example 7

### 2-[(Benzo[b]naphtho[2,3-d]thiophen-6-ylmethyl)amino]-2-methyl-1,3-propanediol

7A Benzo[b]naphtho[2,3-d]thiophene-6-carbaldehyde

Using the procedure outlined in example 2B benzo[b]naphtho[2,3-d]-thiophene(Cambridge Chemical, Inc. gave benzo[b]naphtho]2,3-d]-thiophene-6-carbaldehyde, mp 199° in 80.1% yield which analyzed correctly (C,H,S) for the assigned structure.

7B 2-[(Benzo[b]naphtho[2,3-d]thiophen-6-ylmethyl)amino-2-methyl-1,3-propanediol methanesulfonate

Using the procedure outlined in example 1, benzo[b]naphtho[2,3-d]-thiophene-6-carbaldehyde (7A) and 2-amino-2-methyl-1,3-propanediol (Aldrich) gave 2-[(benzo[b]naphtho[2,3-d]thiophen-6-yl-methyl)amino]-2-methyl-1,3-propanediol methanesulfonate, mp 242-243° in 67.1% yield which analyzed correctly (C,H,N,S) for the assigned structure.

## EXAMPLE 8

### 2-[(Benzo[b]naphtho[2,3-d]thiophen-8-ylmethyl)amino-2-methyl-1,3-propanediol

8A Benzo(b]naphtho[2,3-d]thiophene-8-carbaldehyde

To a round bottom flask equipped with magnetic stirring bar, reflux condenser and $N_2$ inlet line was added 2,3-dichloro-5,6-dicyano-1,4-benzoquinone (DDQ) (Aldrich, 38.6g, 0.119 mol), $H_20$ (100 mL) and $CHC1_3$(1500mL). After refluxing the mixture For 15 minutes 8-methylbenzo[b]naphtho-[2,3-d]thiophene (Cambridge Chemicals, Inc., 21.0g, 89.6 mmol) was added to the flask. The mixture was refluxed for 5h then an additional portion of DDQ (19.3g, 85 mmol) was added. The mixture was then refluxed overnight, cooled and the deep red solution filtered. The solvent was then removed by rotary evaporation and the residual $H_20$ removed by azeotropic distillation with several portions of toluene. The material was the dissolved in toluene (500 mL) and applied to a 40x10 cm column of $SiO_2$ and eluted with additional toluene as the solvent. The appropriate fractions were combined and the solvent removed to give 7.12g of crude material. This was crystallized twice from toluene, filtered and dried to give 5.35g of benzo[b]-naphtho[2,3-d]thiophene-8-carbaldehyde, mp 182-185° (22.7% yield) which analyzed correctly (C,H,S) for the assigned structure.

8B 2-[(Benzo[b]naphtho[2,3-d]thiophen-8-ylmethyl)amino-2-methyl-1,3-propanediol methanesulfonate 6/10 hydrate

Using procedure outlined in example 1, benzo[b]naphtho[2,3-d]-thiophene-8-carbaldehyde (8A) and 2-amino-2-methyl-1,3-propanediol (Aldrich) gave 2-[(benzo[b]naphtho[2,3-d]thiophen-8-yl methyl)amino]-2-methyl-1,3-propanediol methanesulfonate 6/10 hydrate, mp 245-246° (dec) in 60.1% yield which analyzed correctly (C,H,N,S) for the assigned structure.

EXAMPLE 9

2-[(Benzo[b]naphtho[2,3-d]thiophen-7-ylmethyl)amino]-2-methyl-1,3-propanediol

9A Benzo[b]naphtho[2,3-d]thiophene-7-carbaldehyde
Using the procedure described in example 8, 7-methylbenzo[b]-naphtho-[2,3-d]thiophene (Cambridge Chemicals, Inc.) gave benzo[b]naphtho[2,3-d]thiophene-7-carbaldehyde, mp 199-200° in 18.7% yield which analyzed correctly for the assigned structure.
9B 2-[(Benzol[b]naphtho[2,3-d]thiophen-7-ylmethyl)amino]-2-methyl-1,3-propanediol methanesulfonate 1/2 hydrate
Using the procedure outlined in example 1, benzo[b]naphtho[2,3-d]-thiophene-7-carbaldehyde (9A) and 2-amino-2-methyl-1,3-propanediol (Aldrich) gave 2-[(benzo[b]naphtho[2,3-d]-thiophen-7-ylmethyl)amino]-2-methyl-1,3-propanediol methanesulfonate 1/2 hydrate, mp 210-211° (dec) in 75.8% yield which analyzed correctly (C,H,N,S) for the assigned structure

EXAMPLE 10

2[(Benzo[b]naphtho[2,3-d]furan-11-ylmethyl)amino]-2-methyl-1,3-propanediol methanesufonate

Using the procedure outlined in example 1, benzo[b]naphtho[2,3-d]-furan-11-carbaldehyde (2B) and 2-amino-2-methyl-1,3-propanediol (Aldrich) gave 2-[(benzo[b]naphtho[2,3-d]furan-11-ylmethyl)-amino]-2-methyl-1,3-propanediol methanesulfonate, mp 219-220° (dec) in 68.8% yield which analyzed correctly (C,H,N,S) for the assigned structure.

EXAMPLE 11

2-[(5-Ethyl-5H-benzo[b]carbazol-7-yl)methylamino]-2-methyl-1,3-propanediol

11A 5-Ethyl-5H-benzo[b]carbazole-7-carbaldehyde
Using the procedure outlined in example 8A, 5-ethyl-7-methyl-5H-benzo[b]carbazole (Cambridge Chemicals, Inc.) gave 5-ethyl-5H-benzo[b]carbazole-7-carbaldehyde, mp 130-133° in 15.4% yield which analyzed correctly (C,H,N) for the assigned structure.
11B 2-[(5-Ethyl-5H-benzo[b]carbazol-7-yl)methylamino]-2-methyl-1,3-propanediol methanesulfonate
Using the procedure outlined in example 1, 5-ethyl-5H-benzo[b]carbazole-7-carbaldehyde (11A) and 2-amino-2-methyl-1,3-propanediol (Aldrich) gave 2-[(5-ethyl-5H-benzo[b]carbazol-7-yl)-methy]amino]-2-methyl-1,3-propanediol methanesulfonate, mp 219-220° (dec) in 40.2% yield which analyzed correctly (C,H,N,S) for the assigned structure.

EXAMPLE 12

2-[(5-Ethy-5H-benzo[b]carbazol-6-yl)methylamino]-2-methyl-1,3-propanediol

12A 5-Ethyl-5H-benzo[b]carbazole-6-carbaldehyde
Using the procedure outlined in example 2, 5-ethyl-6-methyl-5H-benzo[b]carbazole (Cambridge Chemicals, Inc.) gave 5-ethyl-5H-benzo[b]carbazole-6-carbaldehyde, mp 95.5-96.5° in 44.9% yield which analyzed correctly (C,H,N) for the assigned structure.
12B 2-[(5-Ethyl-5H-benzo[b]carbazol-6-yl)methylamino]-2-methyl-1,3-propanediol methanesulfonate
Using the procedure outlined in example 1, 5-ethyl-5H-benzo[b]carbazole-6-carbaldehyde (12A) and 2-amino-2-methyl-1,3-propanediol (Aldrich) gave 2-[(5-ethyl-5H-benzo[b]carbazol-6-yl)methylamino]-2-methyl-1,3-propanediol methanesulfonate, mp 174-175° in 68.0% yield which analyzed correctly (C,H,N,S) for the assigned structure.

EXAMPLE 13

2-[(Benzo[b]naphtho[1,2-d]thiophen-5-ylmethyl)amino]-2-methy-1,3-propanediol

13A Benzo[b]naphtho[1,2-d]thiophen-5-carbaldehyde
Using the procedure outlined in example 2, benzo[b]naphtho[1,2-d]-thiophene(H.G. Pars Pharmaceutical

Laboratories, Inc.) gave benzo[b]naphtho[1,2-d]thiophen-5-carbaldehyde, mp 142-144° in 43.3% yield which analyzed correctly (C,H,S) for the assigned structure.

13B 2-[(Benzo[b]naphtho[1,2-d]thiophen-5-ylmethyl)amino]-2-methyl-1,3-propanediol methanesulfonate 1/2 hydrate

Using the procedure outlined in example 1, benzo[b]naphtho[1,2-d]-thiophen-5-carbaldehyde (13A) and 2-amino-2-methyl-1,3-propanediol (Aldrich) gave 2-[(benzo[b]naphtho[1,2-d]-thiophen-5-ylmethyl)amino]-2-methyl-1,3-propanediol methanesulfonate 1/2 hydrate, mp 209-209.5° in 73.7% yield which analyzed correctly (C,H,N,S) for the assigned structure.

EXAMPLE 14

2-Methyl-2-[(phenanthro[1,2-b]furan-2-ylmethyl)-amino]-1,3-propanediol

14A Phenanthro[1,2-b]furan-2-methanol

To a round bottom flask equipped with magnetic stirring bar, reflux condenser, nitrogen inlet tube and bubbler was added ethyl phenanthro[1,2-b]furan-2-carboxylate (H.G. Pars Pharmaceutical Laboratories, Inc., 7.9g, 27.2 mmol), lithium borohydride (Aldrich, 0.65g, 30 mmol) and dry THF (400 mL). The mixture was stirred at reflux for 6h and then poured into $H_2O$ (1L). The reaction mixture was acidified with 1N HC1 and the resulting white solid was filtered, washed with additional $H_2O$ (500 mL) then dissolved in $CH_2C1_2$ (500 mL), dried ($Na_2SO_4$), filtered, concentrated to 200 mL and diluted to 500 mL with hexane. The resulting material was filtered, washed with hexane (100 mL) and placed in a vacuum oven overnight. A total of 6.1g of phenanthro[1,2-b]furan-2-methanol, mp 125-126° was obtained which analyzed correctly (C,H) for the assigned structure.

14B Phenanthro[1,2-b]furan-2-carbaldehyde

To a round bottom flask equipped with magnetic stirring bar, reflux condenser, nitrogen inlet tube and bubbler was added phenanthro[1,2-b]furan-2-methanol (14A, 5.84g, 23.5 mmol), barium manganate (Aldrich, 12.06g, 47 mmol) and dry $CH_2C1_2$ (400 mL). The mixture was refluxed for 6h, filtered and the resulting dark yellow solution filtered through a small plug of silica gel to remove inorganic salts and baseline material. The solvent was then removed by rotary evaporation and the crude material recrystallized using $CH_2C1_2$/ hexane to give after drying 5.27g (91% yield) of phenanthro[1,2-b]furan-2-carbaldehyde, mp 169° which analyzed correctly (C,H) for the assigned structure.

14C 2-Methyl-2[(phenanthro[1,2-b]furan-2-ylmethyl)amino-1,3-propanediol methanesulfonate 1/2 hydrate

Using the procedure outlined in example 1, phenanthro[1,2-b]furan-2-carbaldehyde (14B) and 2-amino-2-methyl-1,3-propanediol (Aldrich) gave 2-methyl-2-[(phenanthro[1,2-b]furan-2-ylmethyl)amino]-1,3-propanediol methanesulfonate 1/2 hydrate, mp 168-170° (dec) in 57.1% yield which analyzed correctly (C,H,N,S) for the assigned structure.

EXAMPLE 15

2-Methyl-2-[(phenanthro[1,2-b]furan-11-ylmethyl)amino]-1,3-propanediol

15A Ethyl 11-formyl-phenanthro[1,2-b]furan-2-carboxylate

Using the procedure outlined in example 2B, ethyl phenanthro[1,2-b]-furan-2-carboxylate (H.G.Pars Pharmaceutical Laboratories, Inc.) gave a crude mixture of aldehydes in 54% yield which was used in the next step without purification. An analytical sample of the main component of this mixture, ethyl 11-formyl-phenanthro[1,2-b]furan-2-carboxylate mp *** was produced by column chromotography followed by crystallization ($CH_2C1_2$/hexane).

15B Phenanthro[1,2-b]furan-11-carbaldehyde

To a round bottom flask equipped with magnetic stirring bar, condenser, and nitrogen inlet tube and bubbler was added ethyl 11-formyl-phenanthro[1,2-b]furan-2-carboxylate (15A, 2.5g, 7.8 mmol), 1N sodium hydroxide solution (25 mL), THF (50 mL) and $H_2O$ (25 mL). The mixture was refluxed for 2h until the reaction was homogeneous. The mixture was acidified with 1N HC1 and the solvent removed by rotary evaporation. The crude solid was then heated to 150° with copper powder (0.9g) and quinoline (Aldrich, 25 mL) for 1h. The reaction mixture was cooled and the quinoline removed under vacuum to give a crude dark green solid. After chromatography and crystallization ($CH_2C1_2$/ hexane) 0.71g (37% yield) of phenanthro[1,2-b]furan-11-carbaldehyde, mp 145-150° was obtained which analyzed correctly (C,H) for the assigned structure.

15C 2-Methyl-2-[(phenanthro[1,2-b]furan-4-ylmethyl)amino]-1,3-propandiol methanesulfonate
Using the procedure outlined in example 1, phenanthro[1,2-b]furan-11-carbaldehyde (15B) and 2-amino-2-methyl-1,3-propanediol (Aldrich) gave 2-methyl-2-[(phenanthro[1,2-b]furan-11-ylmethyl)-amino]-1,3-propanediol methanesulfonate, mp 186-188° (dec) in 57.6% yield which analyzed correctly (C,H,N,S) for the assigned structure.

EXAMPLE 16

2-Methyl-2-[(phenanthro[1,2-b]thiophen-2-ylmethyl)amino]-1,3-propanediol

16A Phenanthro[1,2-b]thiophen-2-methanol
Using the procedure outlined in 14A, ethyl phenanthro[1,2-b]thiophen-2-carboxylate (H.G. Pars Pharmaceutical Laboratories, Inc.) gave phenanthro[1,2-b]thiophen-2-methanol, mp 169-170.5° in 98.0% yield which analyzed correctly (C,H,S) for the assigned structure.
16B Phenanthro[1,2-b]thiophene-2-carbaldehyde
Using the procedure outlined in 14B, phenanthro[1,2-b]thiophene-2-methanol (16A) gave phenanthro[1,2-b]thiophene-2-carbaldehyde. mp 209-210° in 82.9% yield which analyzed correctly (C,H,S) for the assigned structure.
16C 2-Methyl-2-[(phenanthro[1,2-b]thiophen-2-ylmethyl)amino]-1,3-propanediol methanesulfonate 3/5 hydrate
Using the procedure outlined in example 1, phenanthro[1,2-b]-thiophene-2-carbaldehyde (16B) and 2-amino-2-methyl-1,3-propanediol (Aldrich) gave 2-methyl-2-[(phenanthro[1,2-b]-thiophen-2-yl]methyl)-amino-1,3-propanediol methanesulfonate 3/5 hydrate, mp 209-209.5° (dec) in 82.7% yield which analyzed correctly (C,H,N,S) for the assigned structure.

EXAMPLE 17

2-Methyl-2-[(phenanthro[1,2-b]thiophen-11-ylmethyl)amino]-1,3-propanediol

17A Phenanthro[1,2-b]thiophene-11-carbaldehyde
Using the procedure outlined in example 2B, ethyl phenanthro[1,2-b]-thiophene-2-carboxylate was formylated to give a low yield of a mixture of aldehyde esters. The mixture was directly hydrolyzed as in example 15B and the resulting crude mixture of aldehyde acids was decarboxylated as in example 15B to give a crude mixture of aldehydes. The main component of the mixture, phenanthro[1,2-b]-thiophene-11-carbaldehyde, mp 161.5-162.5° was obtained after chromatography and crystallization in 3.1% overall yield for the sequence and analyzed correctly (C,H,S) for the assigned structure.
17B 2-Methyl-2-[(phenanthro[1,2-b]thiophen-11-ylmethyl)amino-1,3-propanediol methanesulfonate
Using the procedure outlined in example 1, phenanthro[1,2-b]-thiophene-11-carbaldehyde (17B) and 2-amino-2-methyl-1,3-propanediol (Aldrich) gave 2-methyl-2-[(phenanthro[1,2-b]thiophen-11-ylmethyl)-amino]-1,3-propanediol methanesulfonate, mp 206-207° (dec) in 56.5% yield which analyzed correctly (C,H,N,S) for the assigned structure.

EXAMPLE 18

2-Methyl-2-[(phenanthro[4,3-b]furan-2-ylmethyl)amino]-1,3-propanediol

18A Phenanthro[4,3-b]furan-2-methanol
Using the procedure outlined in example 14A, ethyl phenanthro[4,3-b]-furan-2-carboxylate (H.G. Pars Pharmaceutical Laboratories, Inc.) gave a 91% yield of phenanthro[4,3-b]furan-2-methanol, mp 125-126° which analyzed correctly for the assigned structure.
18B Phenanthro[4,3-b]furan-2-carbaldehyde
Using the procedure outlined in example 14B, phenanthro[4,3-b]furan-2-methanol (18A) gave a 91.2% yield of phenanthro[4,3-b]furan-2-carbaldehyde mp 169° which analyzed correctly for the assigned structure.
18C 2-Methyl-2-[(phenanthro[4,3-b]furan-2-ylmethyl)amino]-1,3-propanediol methanesulfonate
Using the procedure outlined in example 1, phenanthro[4,3-b]furan-2-carbaldehyde (18B) and 2-amino-2-methyl-1,3-propanediol (Aldrich) gave 2-methyl-2-[(phenanthro[4,3-b]furan-2-ylmethyl)amino]-1,3-propanediol methanesulfonate, mp 186-188° (dec) in 56.5% yield which analyzed correctly (C,H,N,S) for the

assigned structure.

EXAMPLE 19

2-Methyl-2-[(phenanthro[4,3-b]thiophen-7-ylmethyl)amino]-1,3-propanediol

19A Phenanthro[4,3-b]thiophene-7-carbaldehyde
Using the procedure outlined in example 17A ethyl phenanthro[4,3-b]-thiophene-2-carboxylate (H.G.Pars Pharmaceutical Laboratories, Inc.) gave a 6.7% yield of phenanthro[4,3-b]thiophene-7-carbaldehyde mp 173-177° which analyzed correctly (C,H.S) for the assigned structure.
19B 2-Methyl-2-[(phenanthro[4,3-b]thiophen-7-ylmethyl)amino]-1,3-propanediol methanesulfonate 1/4 hydrate
Using the procedure outlined in example 1, phenanthro[4,3-b]-thiophene-7-carbaldehyde (19A) and 2-amio-2-methyl-1,3-propanediol (Aldrich) gave 2-methyl-2-[(phenanthro[4,3-b]thiophen-7-ylmethyl)amino]-1,3-propanediol methanesulfonate 1/4 hydrate, mp 189-191° (dec) in 53.8% yield which analyzed correctly (C,H,N,S) for the assigned structure.

EXAMPLE 20

2-Methyl-2-[(phenanthro[9,10-b]furan-2-ylmethyl)amino]-1,3-propanediol

20A Using the procedure outlined in example 2, phenanthro[9,10-b]furan (prepared by the procedure of P.Muller and J. Pfyffer, Chimia 38, 79 (1984)) gave, phenanthro[9,10-b]furan-2-carbaldehyde, mp 84-85° in 32.8% yield which analyzed correctly (C,H) for the assigned structure
20B 2-Methyl-2-[(phenanthro[9,10-b]furan-2-ylmethyl)amino]-1,3-propanediol methanesulfonate 1/5 hydrate 1/5 EtOH
Using the procedure outlined in example 1, phenanthro[9,10-b]furan-2-carbaldehyde (20A) and 2-amino-2-methyl-1,3-propanediol (Aldrich) gave 2-methyl-2-[(phenanthro[9,10-b]furan-2-ylmethyl)-amino]-1,3-propanediol methanesulfonate 1/5 hydrate 1/5 EtOH, mp 218-219° (dec) in 39.2% yield which analyzed correctly (C,H,N,S) for the assigned structure.

EXAMPLE 21

2-Methyl-2-[(phenanthro[9,10-c]thiophen-1-ylmethyl)-amino-1,3-propanediol

21A Phenanthro[9,10-c]thiophene-1-carbaldehyde
Using the procedure outlined in example 2, phenanthro[9,10-c]-thiophene (H.G. Pars Pharmaceutical Laboratories, Inc.) gave phenanthro[9,10-c]thiophene-1-carbaldehyde, mp 198-199° in 85.9% yield which analyzed correctly (C,H,S) for the assigned structure.
21B 2-Methyl-2-[(phenanthro[9,10-c]thiophen-1-ylmethyl)aminol]-1,3-propanediol methanesulfonate 1/4 hydrate
Using the procedure outlined in example 1, phenanthro[9,10-c]-thiophene-1-carbaldehyde (21A) and 2-amino-2-methyl-1,3-propanediol (Aldrich) gave 2-methyl-2-[(phenanthro[9,10-c]thiophen-1-ylmethyl)-amino-1,3-propanediol methanesulfonate 1/4 hydrate, mp 180-187° (dec) in 39.2% yield which analyzed correctly (C,H,N,S) for the assigned structure.

Antitumor Screening Results

Methods for evaluating the antitumor activity of these compounds are essentially those used in the Tumor Panel by the Development Therapeutics Program, Division of Cancer Treatment, National Cancer Institute, A. Goldin, et al., Methods in Cancer Research, Vol. XVI, p. 165, Academic Press (1979). Some modifications, in dose level and schedule have been made to increase the testing efficiency.

Lymphocytic Leukemia P388/0 Test

CD2-$F_1$ mice, of the same sex weighing within 20±3g, are used for this test. Control and test animals are injected intraperitoneally with a suspension of $10^6$ viable P388/0 tumor cells on day 0. In each test several dose levels which bracket the $LD_{20}$ for the compound are evaluated; each dose level group contains

6 animals. The test compounds are prepared either in physiologic saline containing 0.05% Tween 80 or distilled water containing 5% dextrose and are administered intraperitoneally on days, 1, 5, and 9 relative to tumor implant. Doses are on a mg/kg basis according to individual animals' body weights. the day of death for each animal is recorded, the median identified for each group and the ratios of median survival time for treated (T)/control (C) groups are calculated. The criterion for activity is T/C x 100 ≧120%. Results of P388/0 testing are summarized in Table I below.

TABLE I

| Compound of Formula | Optimal Dose (mg/kg) | T/C x 100% (Excluding 30 Day Survivors) | $LD_{20}$ mg/Kg | 30 DAY Survivors |
|---|---|---|---|---|
| 1 | 65 | +210 | 35 | 1/6 |
| 2B | 100 | +204 | 125 | 1/6 |
| 3B | 20 | +230 | 10 | 2/6 |
| 4C | 80 | no deaths | 55 | 6/6 |
| 5B | 125 | +255 | 100 | 4/6 |
| 6B | 55 | +210 | 35 | 0/6 |

Formulation Examples

| A. TABLET | |
|---|---|
| Compound of Formula I (as methanesulphonate) | 500.0. mg |
| Pregelatinised Corn Starch | 60.0 mg |
| Sodium Starch Glycolate | 36.0 mg |
| Magnesium Stearate | 4.0 mg |

The Compound of formula (I) is finely ground and intimately mixed with the powdered excipients, pregelatinised corn starch and sodium starch glycolate. The powders are wetted with purified water to form granules. The granules are dried and mixed with the magnesium stearate. The formulation is then compressed into tablets weighing approximately 600 mg each.

| B. TABLET | |
|---|---|
| Compound of formula (I) | 500.0 mg |
| Corn Starch | 70.0 mg |
| Lactose | 83.8 mg |
| Magnesium Stearate | 4.2mg |
| Polyvinylpyrrolidone | 14.0 mg |
| Stearic Acid | 28.0 mg |

The Compound of formula (I) is finely ground and intimately mixed with the powdered excipients, corn starch and lactose. The powders are wetted with a solution of polyvinylpyrrolidone dissolved in purified water and denatured alcohol to form granules. The granules are dried and mixed with the powdered stearic acid and magnesium stearate. The formulation is then compressed into tablets weighing approximately 700 mg each.

| C. CAPSULES | |
|---|---|
| Compound of formula (I) | 500.0 mg |
| Corn Starch | 50.0 mg |
| Magnesium Stearate | 3.0 mg |

The finely divided compound oF formula (I) is mixed with powdered corn starch and wetted with denatured alcohol to densify the powder. The dried powder is mixed with stearic acid and filled into hard-shell gelatin capsules.

| D. SYRUP | |
|---|---|
| Compound of formula (I) | 250.0 mg |
| Ethanol | 250.0 mg |
| Glycerin | 500.0 mg |
| Sucrose | 3,500.0 mg |
| Flavouring Agent | q.s. |
| Colouring Agent | q.s. |
| Presserving Agent | 0.1% |
| Purified Water | q.s. to 5.0 ml |

The Compound of formula (I) is dissolved in the ethanol, glycerin, and a portion of the purified water. The sucrose and preserving agent are dissolved in another portion of hot purified water, and then the colouring agent is added and dissolved. The two solution are mixed and cooled before the flavouring agent

is added. Purified water is added to final volume. The resulting syrup is throughly mixed.

| E. IV INJECTION | |
|---|---|
| Compound of formula (I) (as methanesulphonate) | 5.0 mg |
| Glycerin | q.s. for isotonicity |
| Preservative | 0.1% |
| Hydrochloric Acid or | as needed for |
| Sodium Hydroxide | pH adjustment |
| Water for Injection | q.s. to 1 ml |

The compound of formula (I) and preservatice is added to the glycerin and a portion of the water for injection. The pH is adjusted with hydrochloric acid or sodium hydroxide. Water for injection is added to final volume and solution is complete after thorough mixing. The solution is sterilised by filtration through a 0.22 micrometer membrane filter and aseptically filled into sterile 10 ml ampoules or vials.

**Claims**

**Claims for the following Contracting States : BE, CH, DE, FR, GB, IT, LI, LU, NL, SE**

**1.** A compound of the formula (I):

$ArCH_2NHR^1$ (I)

or a monomethyl or monoethyl ether thereof, the compound of formula (I) including the said ethers containing no more than 29 carbon atoms in total; an ester thereof; a salt thereof;

wherein Ar is a 6,6,6,5 tetracyclic aromatic ring system having 15 to 17 ring atoms and containing one five membered ring in which there is one heteroatom, optionally substituted by one or two substituents, said substituents containing not more than four carbon atoms in total when taken together and being the same or different and are selected from halogen; cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(0)_nR^2$ wherein n is an integer 0, 1 or 2 and $R^2$ is $C_{1-2}$ alkyl optionally substituted by hydroxy or $C_{1-2}$ alkoxy; or Ar is optionally substituted by a group $NR^3R^4$ containing not more than 5 carbon atoms wherein $R^3$ and $R^4$ are the same or different and each is a $C_{1-3}$ alkyl group or $NR^3R^4$ forms a five or six-membered heterocyclic ring optionally containing one or two additional heteroatoms;

wherein $R^1$ is

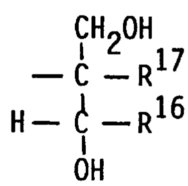

wherein $R^{16}$ is hydrogen or methyl and $R^{17}$ is hydrogen, methyl or ethyl.

**2.** A compound according to claim 1 wherein the tetracyclic aromatic ring system is chosen from the group comprising:

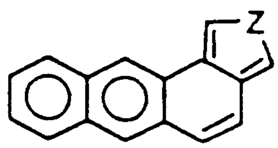

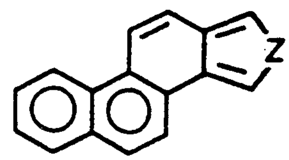

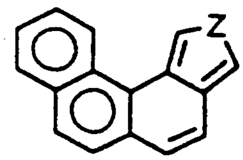

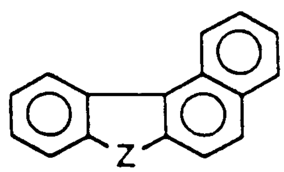

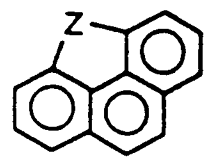

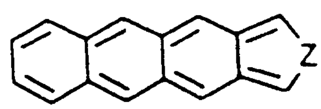

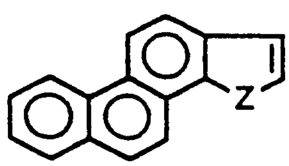

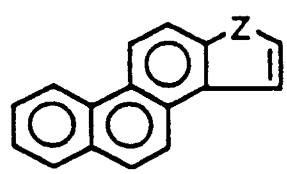

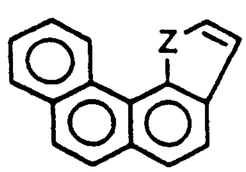

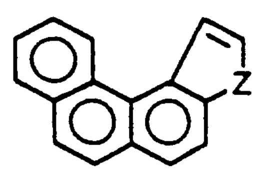

Z is a heteroatom

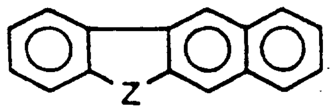

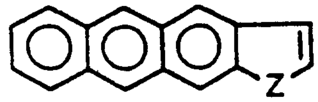

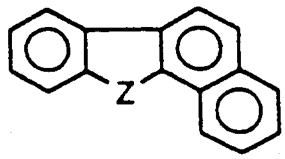

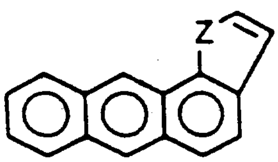

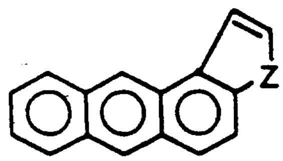

Z is a heteroatom

3. A compound according to claim 2 wherein Ar is selected from:

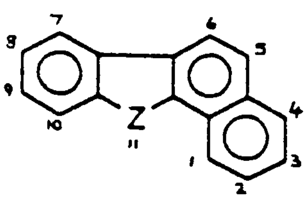

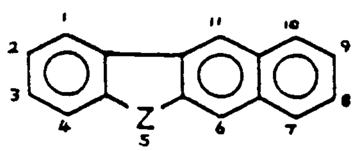

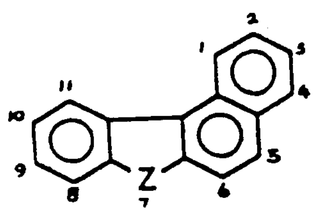

4. A compound according to claim 3 wherein the $CH_2NHR^1$ side chain is attached at one of the positions indicated below:

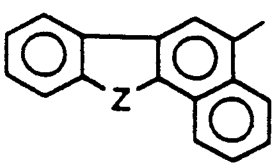

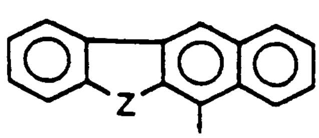

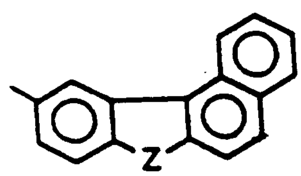

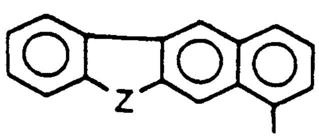

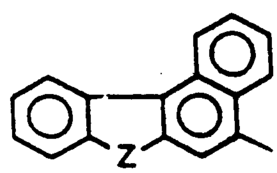

**5.** A compound according to claim 1 wherein Ar is selected from the group comprising:

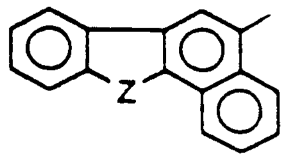

wherein Z = O,S NMe or NEt

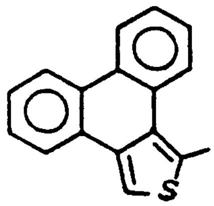

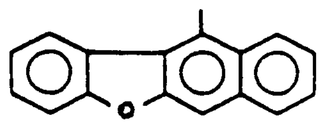

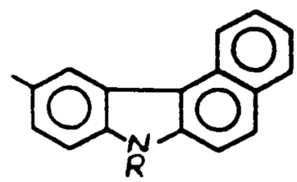

wherein R = Me or Et

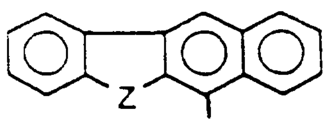

or

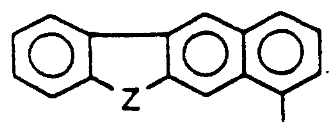

wherein Z = S or O and

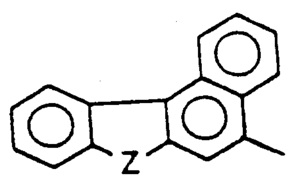

wherein Z = S,O, NMe, or NEt.

**6.** A compound of the formula (I) according to claim 1 in which Ar is:

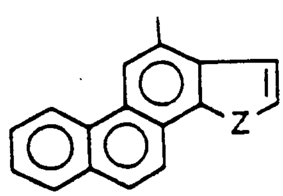

wherein Z = S,O, NMe, or NEt.

**7.** A compound according to claim 1 wherein Ar is 7H-benzo(c)carbazol-10-yl or benzo(b)naphtho(2,1-d)-furan-5-yl.

**8.** 2-[(Benso(b)naphtho(2,1-d]thiophen-5-ylmethyl)amino]-2-methyl-1,3-propanediol
2-[(Benzo(b)naphtho[2,3-d]furan-6-ylmethyl)amino]-2-methyl-1,3-propanediol

2-[(Benzo(b)naphtho[1,2-d]furan-5-ylmethyl)amino]-2-methyl-1,3-propanediol
2-[(Benzo(b)naphtho[2,1-d]furan-5-ylmethyl)amino]-2-methyl-1,3-propanediol
2-Methyl-2-[(7-methyl-7H-benzo[c]carbazol-10-yl)methyl)amino]-1,3-propanediol
2((Benzo[b]naphtho[2,3-d]thiophen-6-ylmethyl)amino)-2-methyl-1,3-propanediol
2-(5-Ethyl-(benzo[b]carbazol-7-ylmethyl)amino)-2-methyl-1,3-propanediol
2- (Benzo[b]naphthol[2,3-d]furan-11-ylmethyl)amino)-2-methyl-1,3-propanediol
2-(Benzo[b]naphtho[2,3-d]thiophen-8-ylmethyl)amino)-2-methyl-1,3-propanediol
2-((5-Ethyl)-benzo[b]carbazol-6-ylmethyl)amino)-2-methyl-1,3-propanediol
2-((Benzo[b]naphtho[2,3-d]thiophen-7-ylmethyl)amino)-2-methyl-1,-3-propanediol
2-((Phenanthro[9,10-c]thiophen-1-ylmethyl)amino)-2-methyl-1,3-propanediol
2-Methyl-2-[(phenanthro[9,10-b]furan-2-ylmethyl)amino]-1,3-propanediol
2-[(Benzo[b]naphtho[1,2-d]thiophen-5-ylmethyl)amino]-2-methyl-1,3-propanediol
2-Methyl-2-[(phenanthro[4,3-b]furan-2-ylmethyl)amino],1,3-propanediol
2-[(Phenanthro[1,2-b]thiophen-2-ylmethyl)amino]-2-methyl-1,3-propanediol
2-Methyl-2-[(phenanthro[1,2-b]furan-2-ylmethyl)amino]-1,3-propanediol
2-Methyl-2-[(phenanthro[4,3-b]thiophen-7-ylmethyl)amino]-1,3-propanediol
2-[(Phenanthro[1,2-b]thiophen-4-ylmethyl)amino]-2-methyl-1,3-propanediol
2-[(Phenanthro[1,2-b]furan-4-ylmethyl)amino]-2-methyl-1,3-propanediol
2-[Benzo[b]naphthol[2,3-d]furan-7-ylmethyl)amino]-2-methyl-1,3-propanediol
and monomethyl ethers, monoethyl ethers, esters and acid addition salts thereof.

**9.** A pharmaceutical formulation comprising a compound as defined in any of claims 1 to 8, and a pharmaceutically acceptable carrier therefor.

**10.** A process for the preparation of a compound of the formula (I), as defined in claim 1, which process comprises:
(a) the reduction of a compound Ar $CH=NR^1$ or an appropriately protected derivative thereof, followed by deprotection where appropriate;
(b) the reduction of a compound $Ar.CO.NHR^1$, wherein the hydroxy groups are optionally protected, followed by deprotection of the hydroxy groups where appropriate;
(c) the reaction of a compound Ar $CH_2L$, wherein L is a leaving group, with a compound $NH_2R^1$;

**11.** Novel chemical intermediates of the formula Ar $CH=NR^1$ wherein Ar and $R^1$ are as defined in claim 1.

**12.** Novel chemechanical intermediates of the formula $Ar.CO.NHR^1$ wherein Ar and $R^1$ are as defined in claim 1.

**13.** Novel chemical intermediates of the formula $ArCH_2L$ wherein Ar is as defined in claim 1 and L is a halogen or sulfonic acid derivative.

**14.** A compound of the formula (I) or an ether, ester or acid addition salt thereof, as defined in claim 1, for use in the treatment of tumours.

**15.** A compound of the formula (I) or an ether, ester or acid addition salt thereof, as defined in claim 1, for use in the preparation of a medicament for the treatment of tumours.

**Claims for the following Contracting State : AT**

**1.** A process for the preparation of a compound of the formula (I):

$ArCH_2NHR^1$ (I)

or a monomethyl or monoethyl ether thereof, the compound of formula (I) including the said ethers containing no more than 29 carbon atoms in total; an ester thereof; a salt thereof;

wherein Ar is a 6,6,6,5 tetracyclic aromatic ring system having 15 to 17 ring atoms and containing one five membered ring in which there is one heteroatom, optionally substituted by one or two substituents, said substituents containing not more than four carbon atoms in total when taken together and being

the same or different and are selected from halogen; cyano; $C_{1-4}$ alkyl or $C_{1-4}$ alkoxy, each optionally substituted by hydroxy or $C_{1-2}$ alkoxy; halogen substituted $C_{1-2}$ alkyl or $C_{1-2}$ alkoxy; a group $S(O)_nR^2$ wherein n is an integer 0, 1 or 2 and $R^2$ is $C_{1-2}$ alkyl optionally substituted by hydroxy or $C_{1-2}$ alkoxy; or Ar is optionally substituted by a group $NR^3R^4$ containing not more than 5 carbon atoms wherein $R^3$ and $R^4$ are the same or different and each is a $C_{1-3}$ alkyl group or $NR^3R^4$ forms a five or six-membered heterocyclic ring optionally containing one or two additional heteroatoms;

wherein $R^1$ is

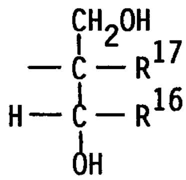

wherein $R^{16}$ is hydrogen or methyl and $R^{17}$ is hydrogen, methyl or ethyl, which process comprises:

(a) the reduction of a compound ArCH-$NR^1$; or an appropriately protected derivative thereof, followed by deprotection where appropriate;

(b) the reduction of a compound Ar. CO. $NHR^1$, wherein the hydroxy groups are optionally protected, followed by deprotection of the hydroxy groups where appropriate;

(c) the reaction of a compound $ArCH_2L$, wherein L is a leaving group, with a compound $NH_2R^1$;

(d) any method known in the art for the preparation of analogous compounds.

**2.** A process according to claim 1 for the preparation of a compound wherein the aromatic ring system is selected from the group comprising:

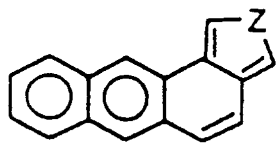

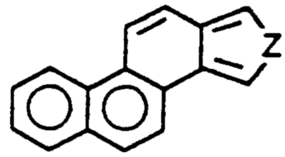

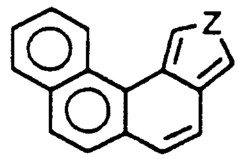

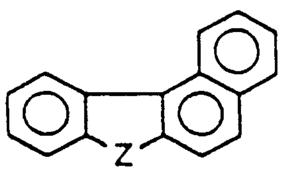

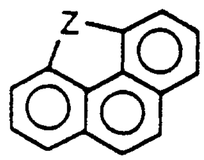

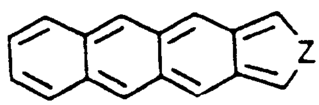

Z is a heteroatom

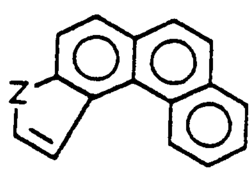

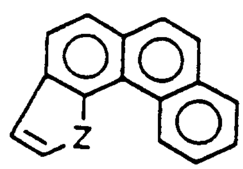

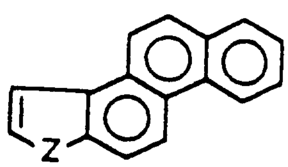

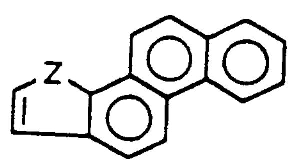

z is a heteroatom

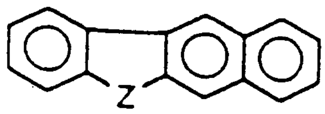

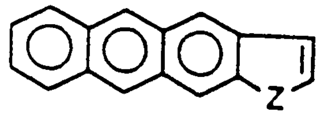

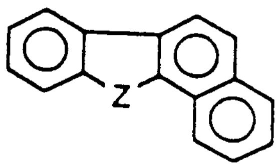

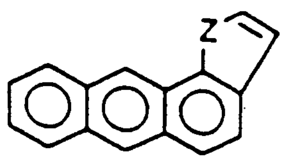

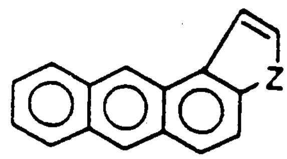

Z is a heteroatom

3. A process according to claim 1 for preparing a compound of the formula (I) wherein Ar is selected from:

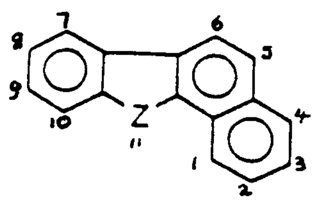

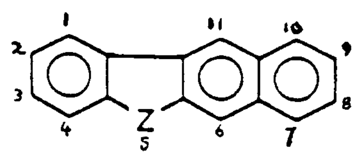

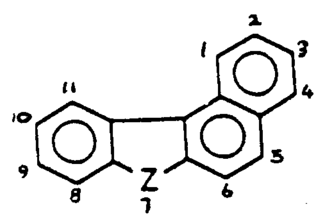

4. A process according to claim 3 for preparing a compound wherein the $CH_2NHR_1$ side chain is attached at one of the positions indicated below:

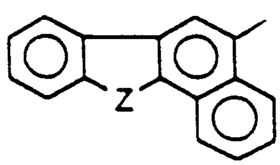

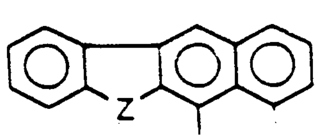

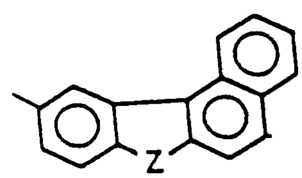

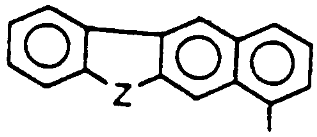

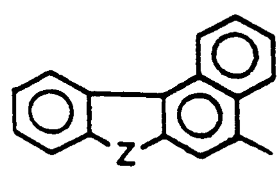

5. A process according to claim 1 for preparing a compound of the formula (I) wherein Ar is selected from:

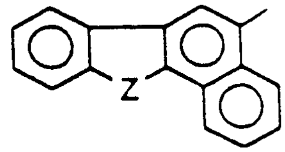

wherein Z = O,S NMe or NEt

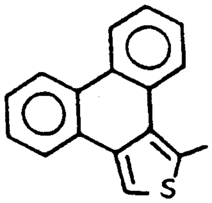

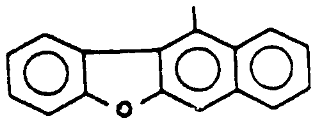

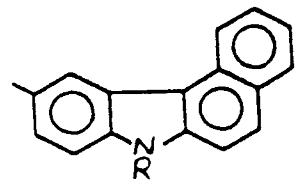

wherein R = Me or Et

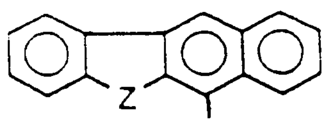

or

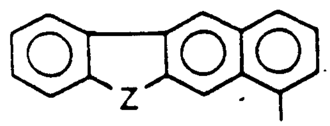

wherein Z = S or O and

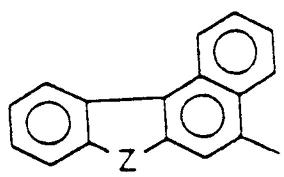

wherein Z = S,O, NMe or NEt.

**6.** A process according to claim 1 for preparing a compound of the formula (I) wherein Ar is:

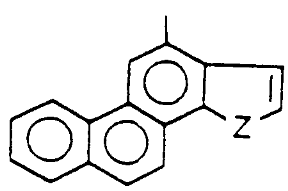

wherein Z = S,O, NMe, or NEt.

**7.** A process according to claim 1 for preparing a compound of the formula (I) wherein Ar is 7H-benzo[c]-carbazol-l0-yl or benzo[b]naphtho[2,1-d]-furan-5-yl.

**8.** A process according to claim 1 for preparing a compound selected from:
2-[(Benzo(b)naphtho(2,1-d]thiophen-5-ylmethyl)amino]-2-methyl-1,3-propanediol
2-[(Benzo(b)naphtho[2,3-d]furan-6-ylmethyl)amino]-2-methyl-1,3-propanediol
2-[(Benzo(b)naphtho[1,2-d]furan-5-ylmethyl)amino]-2-methyl-1,3-propanediol
2-[(Benzo(b)naphtho[2,1-d]furan-5-ylmethyl)amino]-2-methyl-1,3-propanediol

2-Methyl-2-[(7-methyl-7H-benzo[c]carbazol-10-yl)methyl)amino]-1,3-propanediol
2((Benzo[b]naphtho[2,3-d]thiophen-6-ylmethyl)amino)-2-methyl-1,3-propanediol
2-(5-Ethyl-(benzo[b]carbazol-7-ylmethyl)amino)-2-methyl-1,3-propanediol
2-(Benzo(b)naphtho(2,3-d]furan-11-ylmethyl)amino]-2-methyl-1,3-propanediol
2-[(Benzo(b)naphtho(2,3-d]thiophen-8-ylmethyl)amino]-2-methyl-1,3-propanediol
2-((5-Ethyl)-benzo[b]carbazol-6-ylmethyl)amino)-2-methyl-1,3-propanediol
2-(Benzo[b]naphtho[2,3-d]thiophen-7-ylmethyl)amino)-2-methyl-1,-3-propanediol
2-((Phenanthro[9,10-c]thiophen-1-ylmethyl)amino)-2-methyl-1,3-propanediol
2-Methyl-2-[(phenanthro[9,10-b]furan-2-ylmethyl)amino),1,3-propanediol
2-[Benzo[b]naphtho[1,2-d]thiophen-5-ylmethyl)amino]-2-methyl-1,3-propanediol
2-Methyl-2-[(phenanthro[4,3-b]furan-2-ylmethyl)amino]-1,3-propanediol
2-[(Phenanthro[1,2-b]thiophen-2-ylmethyl)amino]-2-methyl-1,3-propanediol
2-Methyl-2-[(phenanthro[1,2-b]furan-2-ylmethyl)amino]-1,3-propanediol
2-Methyl-2-[(phenanthro[4,3-b]thiopen-7-ylmethyl)amino]-1,3-propanediol
2-[(Phenanthro[1,2-b]thiophen-4-ylmethyl)amino]-2-methyl-1,3-propanediol
2-[(Phenanthro[1,2-b]furan-4-ylmethyl)amino]-2-methyl-1,3-propanediol
2-[Benzo[b]naphthol[2,3-d]furan-7-ylmethyl)amino]-2-methyl-1,3-propanediol
and monomethyl ethers, monoethyl ethers, esters and acid addition salts thereof.

**9.** Novel chemical intermediates of the formula $ArCH=NR^1$ wherein Ar and $R^1$ are as defined in claim 1.

**10.** Novel chemical intermediates of the formula $Ar.CO.NHR^1$ wherein Ar and $R^1$ are as defined in claim 1.

**11.** Novel chemical intermediates of the formula $ArCH_2L$ wherein Ar is as defined in claim 1 and L is a halogen or sulfonic acid derivative.

**12.** A pharmaceutical formulation comprising a compound of the formula (I), or an ether, ester or acid addition salt thereof, as defined in claim 1, and a pharmaceutically acceptable carrier therefor.

## Patentansprüche

## Patentansprüche für folgende Vertragsstaaten : BE, CH, DE, FR, GB, IT, LI, LU, NL SE

**1.** Verbindung der Formel (I):

$ArCH_2NHR^1$ (I)

oder ein Monomethyl- oder Monoethylether hiervon, wobei die Verbindung der Formel (I) einschließlich der Ether insgesamt nicht mehr als 29 Kohlenstoffatome enthält; ein Ester hiervon; ein Salz hiervon; worin Ar ein 6,6,6,5-tetracyclisches aromatisches Ringsystem mit 15 bis 17 Ringatomen ist, welches einen fünfgliedrigen Ring enthält, in welchem ein Heteroatom vorliegt, gegebenenfalls substituiert durch einen oder zwei Substituenten, wobei die Substituenten zusammengenommen insgesamt nicht mehr als 4 Kohlenstoffatome enthalten und gleich oder verschieden sind und aus Halogen; Cyano; $C_{1-4}$-Alkyl oder $C_{1-4}$-Alkoxy, die jeweils gegebenenfalls durch Hydroxy oder $C_{1-2}$-Alkoxy substituiert sind: halogensubstituiertes $C_{1-2}$-Alkyl oder $C_{1-2}$-Alkoxy; einer Gruppe $S(O)_nR^2$, worin n eine ganze Zahl aus O, I oder 2 und $R^2$ $C_{1-2}$-Alkyl, das gegebenenfalls durch Hydroxy oder $C_{1-2}$-Alkoxy substituiert ist, bedeuten; ausgewählt sind, oder worin Ar gegebenenfalls durch eine Gruppe $NR^3R^4$ substituiert ist, welche nicht mehr als 5 Kohlenstoffatome enthält, worin $R^3$ und $R^4$ gleich oder veschieden sind und jeweils eine $C_{1-3}$-Alkylgruppe bedeuten oder worin $NR^3R^4$ einen fünf- oder sechsgliedrigen heterocyclischen Ring, der gegebenenfalls ein oder zwei zusätzliche Heteroatome enthält, bildet; und
$R^1$

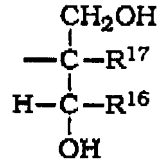

ist, worin $R^{16}$ Wasserstoff oder Methyl und $R^{17}$ Wasserstoff. Methyl oder Ethyl bedeuten.

**2.** Verbindung nach Anspruch 1, wobei das tetracyclische aromatische Ringsystem aus der

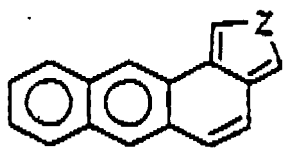

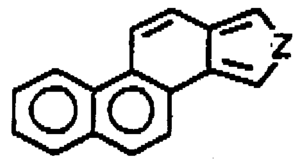

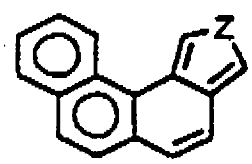

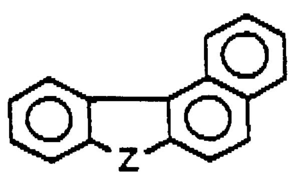

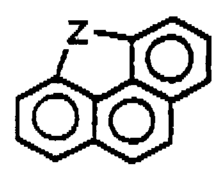

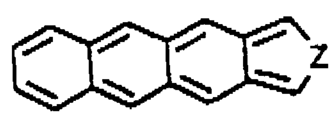

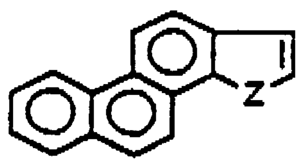

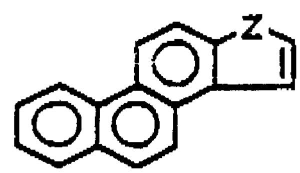

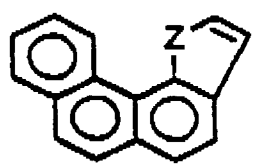

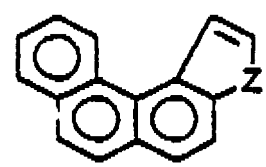

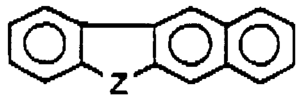

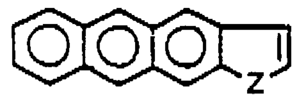

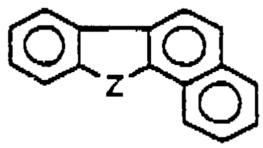

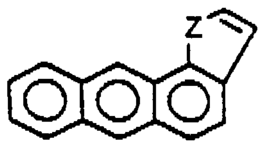

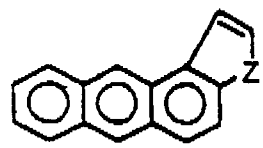

umfassenden Gruppe ausgewählt ist, worin Z ein Heteroatom ist.

**3.** Verbindung nach Anspruch 2, wobei Ar aus

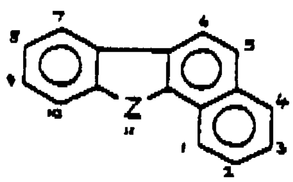

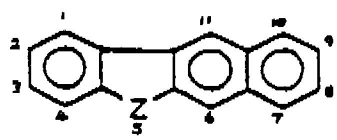

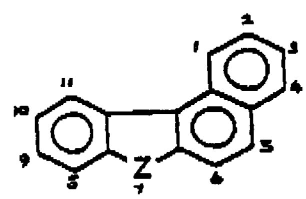

ausgewählt ist.

**4.** Verbindung nach Anspruch 3, wobei die $CH_2NHR^1$-Seitenkette an einer der nachfolgend angezeigten Positionen gebunden ist:

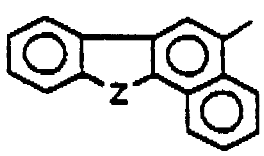

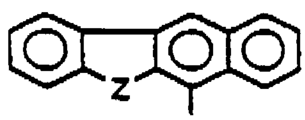

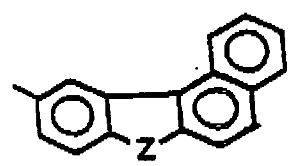

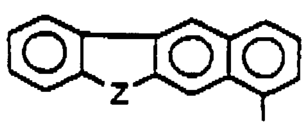

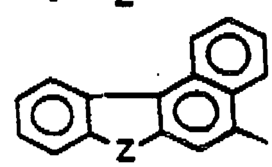

**5.** Verbindung nach Anspruch 1, wobei Ar aus der

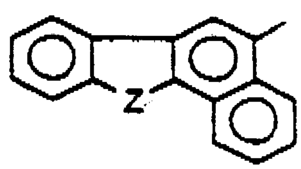

worin Z = O, S NMe oder NEt

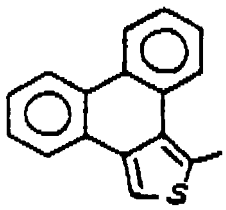

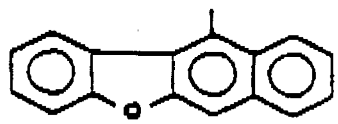

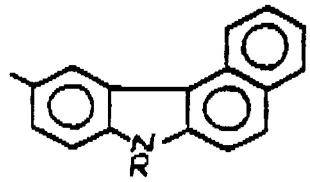

worin R = Me oder Et

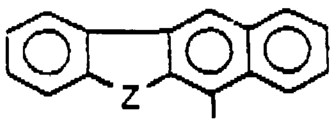

oder

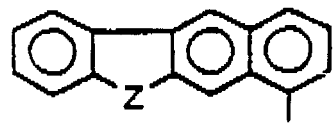

worin Z = S oder O
und

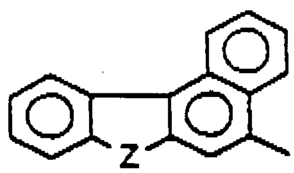

worin Z = S, O, NMe oder NEt
umfassenden Gruppe ausgewählt ist.

6. Verbindung der Formel (I) nach Anspruch 1, wobei Ar

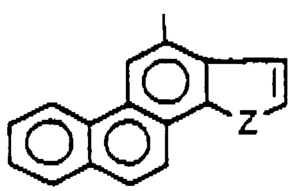

ist,
worin Z = S, O, NMe oder NEt.

7. Verbindung nach Anspruch 1, wobei Ar 7H-Benzo(c)carbazol-10-yl oder Benzo(b)naphtho-[2,1-d)-furan-5-yl ist.

8. 2-[(Benzo(b)naphtho[2,1-d]thiophen-5-ylmethyl)-amino]-2-methyl-1,3-propandiol,
2-[(Benzo(b)naphtho[2,3-d]furan-6-ylmethyl)-amino]-2-methyl-1,3-propandiol,
2-[(Benzo(b)naphtho[1,2-d]furan-5-ylmethyl)-amino]-2-methyl-1,3-propan-diol.
2-[(Benzo(b)naphtho[2,1-d]furan-5-ylmethyl)-amino]-2-methyl-1,3-propandiol,
2-Methyl-2-[(7-methyl-7H-benzo[c]carbazol-10-yl)-methyl)-amino]-1,3-propandiol,
2-((Benzo(b)naphtho[2,3-d]thiophen-6-ylmethyl)-amino)-2-methyl-1,3-propandiol,
2-(5-Ethyl-(benzo[b]carbazol-7-ylmethyl)amino)-2-methyl-1,3-propandiol.
2-(Benzo(b)naphtho[2,3-d]furan-11-ylmethyl)-amino)-2-methyl-1,3-propandiol.
2-(Benzo(b)naphtho[2,3-d]thiophen-8-ylmethyl)-amino)-2-methyl-1,3-propandiol,

2-((5-Ethyl)-benzo[b]carbazol-6-ylmethyl)amino)-2-methyl-1,3-propandiol.
2-((Benzo(b)naphtho[2,3-d]thiophen-7-ylmethyl)-amino)-2-methyl-1,3-propandiol,
2-((Phenanthro[9,10-c]thiophen-1-ylmethyl)-amino)-2-methyl-1,3-propandiol,
2-Methyl-2-[(phenanthro[9,10-b]furan-2-ylmethyl)-amino]-1,3-propandiol,
2-[(Benzo(b)naphtho[1,2-d]thiophen-5-ylmethyl)-amino]-2-methyl-1,3-propandiol,
2-Methyl-2-[(phenanthro[4,3-b]furan-2-ylmethyl)-amino]-1,3-propandiol,
2-[(Phenanthro[1,2-b]thiophen-2-ylmethyl)-amino]-2-methyl-1,3-propandiol,
2-Methyl-2-[(phenanthro[1,2-b]furan-2-ylmethyl)-amino]-1,3-propandiol,
2-Methyl-2-[(phenanthro[4,3-b]thiophen-7-ylmethyl)-amino]-1,3-propandiol,
2-[(Phenanthro[1,2-b]thiophen-4-ylmethyl)-amino]-2-methyl-1,3-propandiol,
2-[(Phenanthro[1,2-b]furan-4-ylmethyl)-amino]-2-methyl-1,3-propandiol,
2-[Benzo[b]naphtho[2,3-d]furan-7-ylmethyl)-amino]-2-methyl-1,3-propandiol
und Monomethylether, Monoethylether, Ester und Säureadditionssalze hiervon.

**9.** Pharmazeutische Zubereitung, umfassend eine Verbindung nach mindestens einem der Ansprüche 1 bis 8 und einen pharmazeutisch annehmbaren Träger hierfür.

**10.** Verfahren zur Herstellung einer Verbindung der Formel (I), wie in Anspruch 1 definiert, welches Verfahren
(a) die Reduktion einer Verbindung Ar $CH=NR^1$ oder eines in geeigneter Weise geschützten Derivats hiervon und, falls zweckmäßig, nachfolgende Entfernung der Schutzgruppe;
[b) die Reduktion einer Verbindung Ar-CO-$NHR^1$, in der die Hydroxygruppen gegebenenfalls geschützt sind, und, falls zweckmäßig, nachfolgende Entfernung der Schutzgruppen von den Hydroxygruppen;
(c) die Umsetzung einer Verbindung Ar $CH_2L$, worin L eine abgehende Gruppe ist, mit einer Verbindung $NH_2R^1$;
umfaßt.

**11.** Neue chemische Zwischenverbindungen der Formel Ar $CH=NR^1$, worin Ar und $R^1$ wie in Anspruch 1 definiert sind.

**12.** Neue chemische Zwischenverbindungen der Formel Ar-CO-$NHR^1$, worin Ar und $R^1$ wie in Anspruch 1 definiert sind.

**13.** Neue chemische Zwischenverbindungen der Formel $ArCH_2L$, worin Ar wie in Anspruch 1 definiert ist und L ein Halogen oder Sulfonsäurederivat ist.

**14.** Verbindung der Formel (I) oder ein Ether, Ester oder Säureadditionssalz hiervon nach Anspruch 1 zur Verwendung bei der Behandlung von Tumoren.

**15.** Verbindung der Formel (I) oder ein Ether, Ester oder Säureadditionssalz hiervon nach Anspruch 1 zur Verwendung bei der Herstellung eines Arzneimittels zur Behandlung von Tumoren.

**Patentanspruch für folgenden Vertragsstaat : AT**

**1.** Verfahren zur Herstellung einer Verbindung der Formel (I):

$ArCH_2NHR^1$ (I)

oder eines Monomethyl- oder Monoethylethers hiervon, wobei die Verbindung der Formel (I) einschließlich der Ether insgesamt nicht mehr als 29 Kohlenstoffatome enthält; eines Esters hiervon; eines Salzes hiervon;
worin Ar 6,6,6,5-tetracyclisches aromatisches Ringsystem mit 15 bis 17 Ringatomen ist, welches einen fünfgliedrigen Ring enthält, in welchem ein Heteroatom vorliegt, gegebenenfalls substituiert durch einen oder zwei Substituenten, wobei die Substituenten zusammengenommen insgesamt nicht mehr als 4 Kohlenstoffatome enthalten und gleich oder verschieden sind und aus Halogen; Cyano; $C_{1-4}$-Alkyl oder $C_{1-4}$-Alkoxy, die jeweils gegebenenfalls durch Hydroxy oder $C_{1-2}$-Alkoxy substituiert sind; halogensubstituiertes $C_{1-2}$-Alkyl oder $C_{1-2}$-Alkoxy; einer Gruppe $S(O)_nR^2$, worin n eine ganze Zahl aus 0, 1 oder 2

und $R^2$ $C_{1-2}$-Alkyl, das gegebenenfalls durch Hydroxy oder $C_{1-2}$-Alkoxy substituiert ist, bedeuten; ausgewählt sind, oder worin Ar gegebenenfalls durch eine Gruppe $NR^3R^4$ substituiert ist, welche nicht mehr als 5 Kohlenstoffatome enthält, worin $R^3$ und $R^4$ gleich oder veschieden sind und jeweils eine $C_{1-3}$-Alkylgruppe bedeuten oder worin $NR^3R^4$ einen fünf- oder sechsgliedrigen heterocyclischen Ring, der gegebenenfalls ein oder zwei zusätzliche Heteroatome enthält, bildet; und
$R^1$

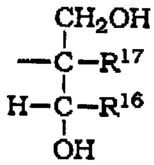

ist, worin $R^{16}$ Wasserstoff oder Methyl und $R^{17}$ Wasserstoff, Methyl oder Ethyl bedeuten,
welches Verfahren

(a) die Reduktion einer Verbindung Ar $CH=NR^1$ oder eines in geeigneter Weise geschützten Derivats hiervon und, falls zweckmäßig, nachfolgende Entfernung der Schutzgruppe;

(b) die Reduktion einer Verbindung Ar•CO•$NHR^1$, in der die Hydroxygruppen gegebenenfalls geschützt sind, und, falls zweckmäßig, nachfolgende Entfernung der Schutzgruppen von den Hydroxygruppen;

(c) die Umsetzung einer Verbindung Ar $CH_2L$, worin L eine abgehende Gruppe ist, mit einer Verbindung $NH_2R^1$;

(d) irgendein im Stand der Technik zur Herstellung analoger Verbindungen bekanntes Verfahren;

umfaßt.

**2.** Verfahren nach Anspruch 1 zur Herstellung einer Verbindung, bei der das aromatische Ringsystem aus der

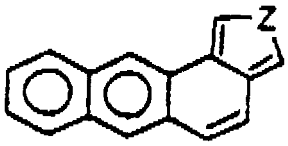

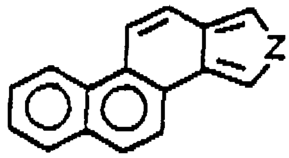

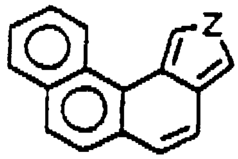

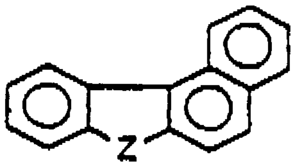

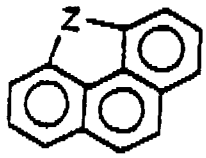

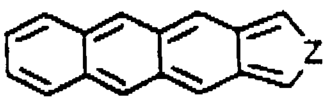

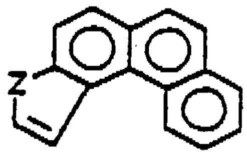

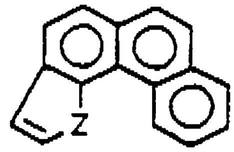

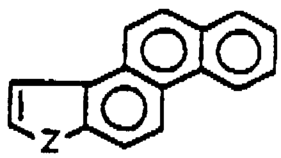

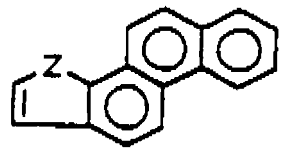

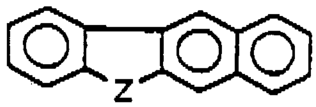

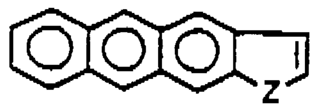

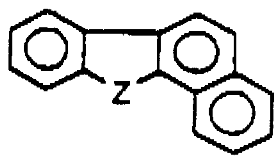

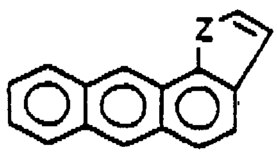

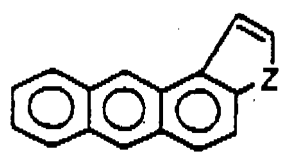

worin Z ein Heteroatom ist, umfassenden Gruppe ausgewählt ist.

**3.** Verfahren nach Anspruch 1 zur Herstellung einer Verbindung der Formel (I), worin Ar aus

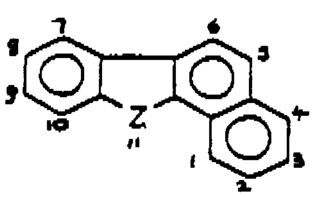

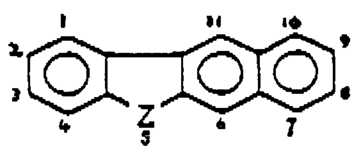

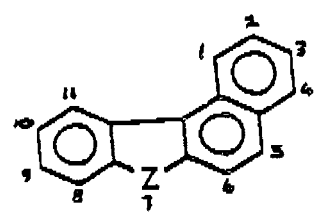

ausgewählt ist.

**4.** Verfahren nach Anspruch 3 zur Herstellung einer Verbindung, bei der die $CH_2NHR^1$-Seitenkette an einer der nachstehend gezeigten Positionen gebunden ist:

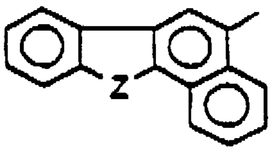
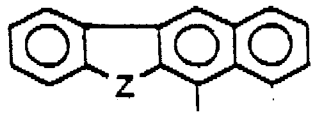
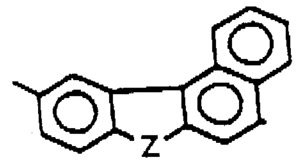
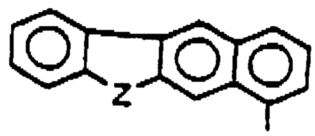
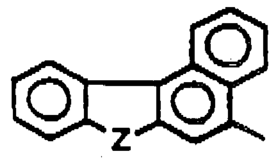

5. Verfahren nach Anspruch 1 zur Herstellung einer Verbindung der Formel (I), worin Ar

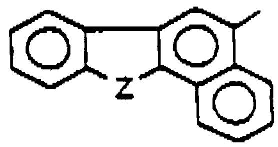

worin Z = O, S NMe oder NEt

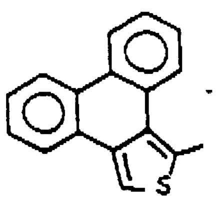
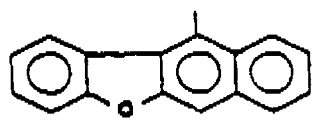
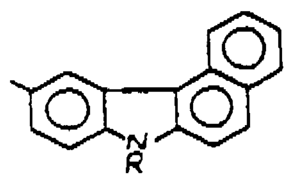

worin R = Me oder Et

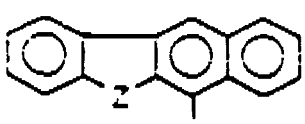

oder

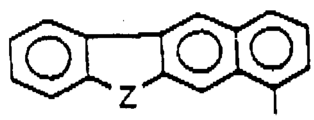

worin Z = S oder O

und

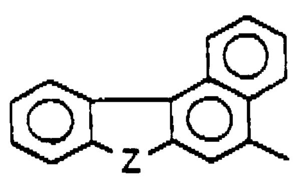

worin Z = S, O, NMe oder NEt

umfassenden Gruppe ausgewählt ist.

6. Verfahren nach Anspruch 1 zur Herstellung einer Verbindung der Formel (I), worin Ar

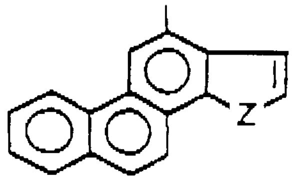

ist, worin Z = S, O, NMe oder NEt.

7. Verfahren nach Anspruch 1 zur Herstellung einer Verbindung der Formel (I), worin Ar 7H-Benzo(c)-carbazol-10-yl oder Benzo(b)naphtho-[2,1-d)-furan-5-yl ist.

8. Verfahren nach Anspruch 1 zur Herstellung einer Verbindung, die aus
2-[(Benzo(b)naphtho[2,1-d]thiophen-5-ylmethyl)-amino]-2-methyl-1,3-propandiol,
2-[(Benzo(b)naphtho[2,3-d]furan-6-ylmethyl)-amino]-2-methyl-1,3-propandiol,
2-[(Benzo(b)naphtho[1,2-d]furan-5-ylmethyl)-amino]-2-methyl-1,3-propandiol,
2-[(Benzo(b)naphtho[2,1-d]furan-5-ylmethyl)-amino]-2-methyl-1,3-propandiol,
2-Methyl-2-[(7-methyl-7H-benzo[c]carbazol-10-yl)-methyl)-amino]-1,3-propandiol.
2-((Benzo(b)naphtho[2,3-d]thiophen-6-ylmethyl)-amino)-2-methyl-1,3-propandiol.
2-(5-Ethyl-[benzo[b]carbazol-7-ylmethyl)amino)-2-methyl-1,3-propandiol,
2-(Benzo(b)naphtho[2,3-d]furan-11-ylmethyl)-amino)-2-methyl-1,3-propandiol,
2-(Benzo(b)naphtho[2,3-d]thiophen-8-ylmethyl)-amino)-2-methyl-1,3-propandiol.
2-((5-Ethyl)-benzo[b]carbazol-6-ylmethyl)amino)-2-methyl-1,3-propandiol,
2-((Benzo(b)naphtho[2-3-d]thiophen-7-ylmethyl)-amino)-2-methyl-1,3-propandiol,
2-((Phenanthro[9,10-c]thiophen-1-ylmethyl)-amino)-2-methyl-1,3-propandiol,
2-Methyl-2-[(phenanthro[9,10-b]furan-2-ylmethyl)-amino]-1,3-propandiol,
2-[(Benzo(b)naphtho[1,2-d]thiophen-5-ylmethyl)-amino]-2-methyl-1,3-propandiol,
2-Methyl-2-[(phenanthro[4,3-b]furan-2-ylmethyl)-amino]-1,3-propandiol,
2-[(Phenanthro[1,2-b]thiophen-2-ylmethyl)-amino]-2-methyl-1,3-propandiol,
2-Methyl-2-[(phenanthro[1,2-b]furan-2-ylmethyl)-amino]-1,3-propandiol,
2-Methyl-2-[(phenanthro[4,3-b]thiophen-7-ylmethyl)-amino]-1,3-propandiol,
2-[(Phenanthro[1,2-b]thiophen-4-ylmethyl)-amino]-2-methyl-1,3-propandiol,
2-[(Phenanthro[1,2-b]furan-4-ylmethyl)-amino]-2-methyl-1,3-propandiol,
2-[Benzo[b]naphtho[2,3-d]furan-7-ylmethyl)-amino]-2-methyl-1,3-propandiol
und Monomethylethern, Monoethylethern. Estern und Säureadditionssalzen hiervon ausgewählt ist.

9. Neue chemische Zwischenverbindungen der Formel Ar CH=NR[1], worin Ar und R[1] wie in Anspruch 1 definiert sind.

10. Neue chemische Zwischenverbindungen der Formel Ar•CO•NHR[1], worin Ar und R[1] wie in Anspruch 1 definiert sind.

11. Neue chemische Zwischenverbindungen der Formel $ArCH_2L$, worin Ar wie in Anspruch 1 definiert ist und L ein Halogen oder Sulfonsäurederivat ist.

12. Pharmazeutische Zubereitung, umfassend eine Verbindung der Formel (I) oder einen Ether, Ester oder ein Säureadditionssalz hiervon, wie in Anspruch 1 definiert, und einen pharmazeutisch annehmbaren Träger hierfür.

**Revendications**
**Revendications pour les Etats contractants suivants : BE, CH, DE, FR, GB, IT LI, LU, NL, SE**

1. Composé de la formule (I):

$ArCH_2NHR^1$ (I)

ou un éther de monométhyle ou de monoéthyle de celui-ci, le composé de la formule (I) y compris lesdits éthers ne contenant pas plus de 29 atomes de carbone au total; un de leurs esters; un de leurs sels;
où Ar est un système cyclique aromatique 6,6,6,5-tétracyclique ayant 15 à 17 atomes de cyclique et contenant un cycle à 5 membres dans lequel il y a un hétéroatome, facultativement substitué par un ou deux substituants, lesdits substituants ne contenant pas plus de 4 atomes de carbone au total quand ils sont pris ensemble et sont les mêmes ou différents et choisis parmi halogène; cyano; $C_{1-4}$-alkyle ou $C_{1-4}$-alkoxy, chacun facultativement substitué par hydroxyle ou $C_{1-2}$-alkoxy; $C_{1-2}$-alkyle ou $C_{1-2}$-alkoxy substitué par halogène; un groupe $S(O)_nR^2$ où n est un entier 0,1 ou 2 et $R^2$ est $C_{1-2}$-alkyle facultativement substitué par hydroxyle ou $C_{1-2}$-alkoxy; ou Ar est facultativement substitué par un groupe $NR^3R^4$ ne contenant pas plus de 5 atomes de carbone, où $R^3$ et $R^4$ sont les mêmes ou différents et chacun est un groupe $C_{1-3}$-alkyle ou $NR_3R^4$ forme un anneau hétérocyclique à 5 ou 6 membres, contenant facultativement 1 ou 2 hétéroatomes additionnels;
$R^1$ est

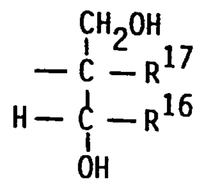

où $R^{16}$ est hydrogène ou méthyle et $R^{17}$ est hydrogène, méthyle ou éthyle.

**2.** Composé selon la revendication 1 où le système aromatique tétracyclique est choisi dans le groupe comprenant:

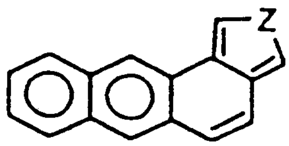

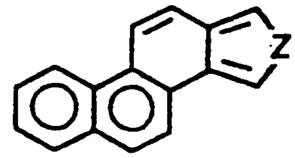

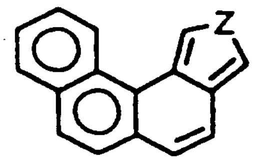

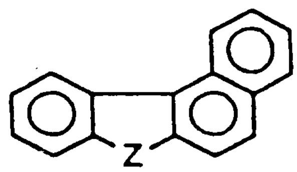

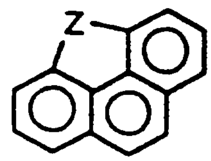

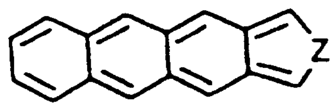

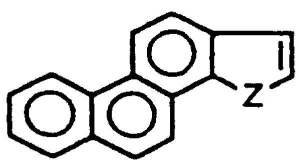

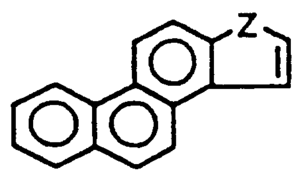

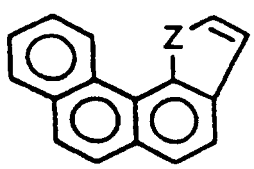

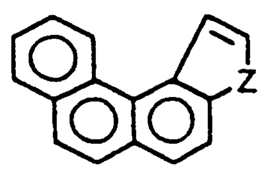

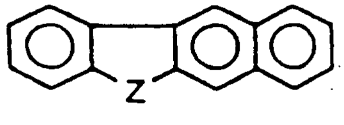

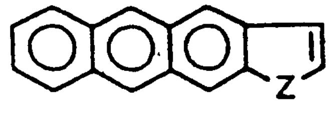

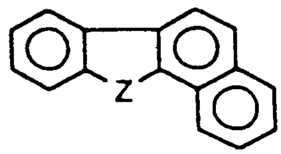

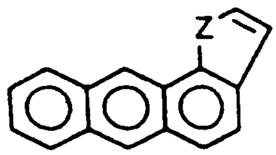

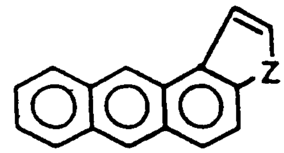

où Z est un hétéroatome.

**3.** Composé selon la revendication 2 où Ar est choisi parmi:

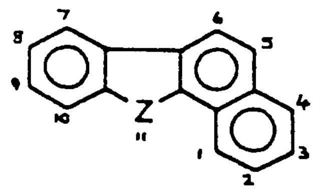

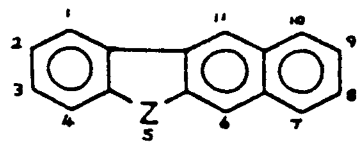

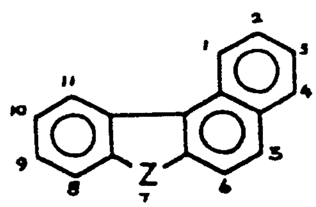

**4.** Composé selon la revendication 3 où la chaîne latérale $CH_2NHR^1$ est attachée à l'une des positions suivantes:

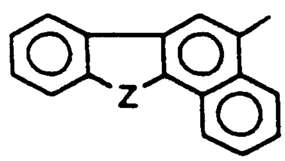

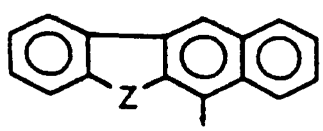

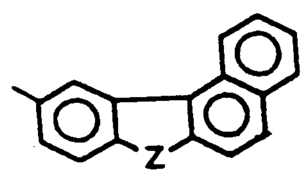

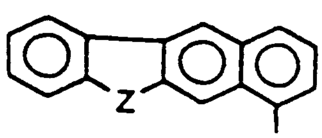

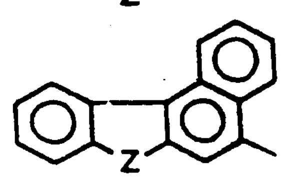

**5.** Composé selon la revendication 1 où Ar est choisi parmi le groupe comprenant:

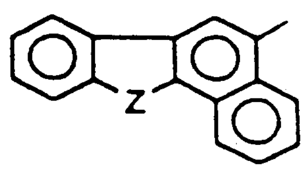

où Z = O, S, NMe ou NEt

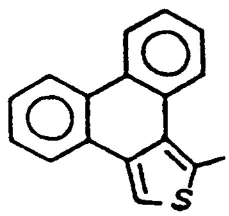

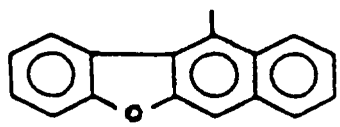

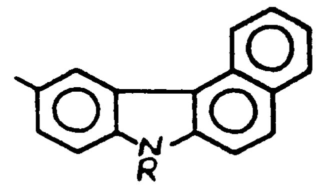

où R = Me ou Et

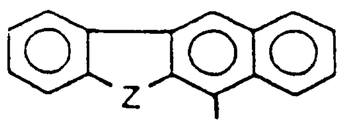

ou

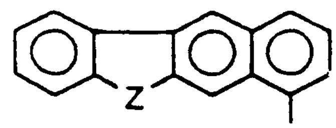

où Z = S ou O, et

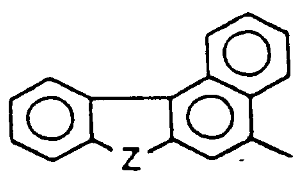

où Z = S, O, NMe, ou NEt

6. Composé de la formule (I) selon la revendication 1 où Ar est:

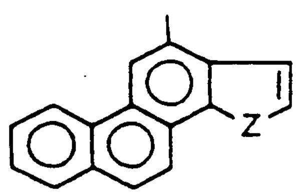

où Z = S, O, NMe, ou NEt

7. Composé selon la revendication 1 où Ar est 7H-benzo(c)carbazol-10-yle le benzo(b)naphto(2,1-d)-furann-5-yle.

8. Composé choisi parmi:
le 2-[(9-benzo[b]naphto[2,1-d]thiophèn-5-ylméthyl)amino]-2-1,3-propanediol
le 2-[(benzo[b]naphto[2,3-d]furann-6-ylméthyl)amino]-2-méthyl-1,3-propanediol
le 2-[(benzo[b]naphto[1,2-d]furann-5-ylméthyl)amino]-2-méthyl-1,3-propanediol
le 2-[(benzo[b]naphto[2,1-d]furann-5-ylméthyl)amino]-2-méthyl-1,3-propanediol
le 2-méthyl-2-[((7-méthyl-7H-benzo[c]carbazol-10-yl)méthyl)amino]-1,3-propanediol
le 2-((benzo[b]naphto[2,3-d]thiophèn-6-ylméthyl)amino)-2-méthyl-1,3-propanediol
le 2-(5-éthyl-(benzo[b]carbazol-7-ylméthyl)amino)-2-méthyl-1,3-propanediol
le 2-((benzo[b]naphto[2,3-d]furann-11-ylméthyl)amino)-2-méthyl-1,3-propanediol
le 2-((benzo[b]naphto[2,3-d]thiophèn-8-ylméthyl)amino)-2-méthyl-1,3-propanediol
le 2-((5-éthyl)-benzo[b]carbazol-6-ylméthyl)amino)-2-méthyl-1,3-propanediol
le 2-((benzo[b]naphto[2,3-d]thiophèn-7-ylméthyl)amino)-2-méthyl-1,3-propanediol
le 2-((phénanthro[9,10-c]thiophèn-1-ylméthyl)amino)-2-méthyl-1,3-propanediol

le 2-méthyl-2-[(phénanthro[9,10-b]furann-2-ylméthyl)amino)-1,3-propanediol
le 2-[(benzo[b]naphto[1,2-d]thiophèn-5-ylméthyl)amino]-2-méthyl-1,3-propanediol
le 2-méthyl-2-[(phénanthro[4,3-b]furann-2-ylméthyl)amino]-1,3-propanediol
le 2-[(phénanthro[1,2-b]thiophèn-2-ylméthyl)amino]-2-méthyl-1,3-propanediol
le 2-méthyl-2-[(phénanthro[1,2-b]furann-2-ylméthyl)amino]-1,3-propanediol
le 2-méthyl-2-[(phénanthro[4,3-b]furann-7-ylméthyl)amino]-1,3-propanediol
le 2-[(phénanthro[1,2-b]thiophèn-4-ylméthyl)amino]-2-méthyl-1,3-propanediol
le 2-[(phénanthro[1,2-b]furann-4-ylméthyl)amino]-2-méthyl-1,3-propanediol
le 2-[(benzo[b]naphto[2,3-d]furann-7-ylméthyl)amino]-2-méthyl-1,3-propanediol
et leurs éthers de monométhyle ou de monoéthyle, esters et sels d'addition d'acides.

**9.** Composition pharmaceutique comprenant un composé tel que défini dans l'une quelconque des revendications 1 à 8 et un excipient pharmaceutiquement acceptable.

**10.** Procédé de préparation d'un composé de la formule (I), tel que défini dans la revendication 1, qui comprend:

(a)- la réduction d'un composé Ar-CH = $NR^1$ ou d'un dérivé protégé de celui-ci, suivie de la déprotection lorsqu'il y a lieu;

(b)- la réduction d'un composé Ar.CO.$NHR^1$, où les groupes hydroxyle sont facultativement protégés, suivie de la déprotection des groupes hydroxyle lorsqu'il y a lieu;

(c)- la réaction d'un composé $ArCH_2L$, où L est un groupe partant, avec un composé $NH_2R^1$.

**11.** Nouveaux intermédiaires chimiques de la formule Ar-CH = $NR^1$, où Ar et $R^1$ sont tels que définis dans la revendication 1.

**12.** Nouveaux intermédiaires chimiques de la formule Ar.CO.$NHR^1$, où Ar et $R^1$ sont tels que définis dans la revendication 1.

**13.** Nouveaux intermédiaires chimiques de la formule $ArCH_2L$, où Ar est tel que défini dans la revendication 1 et L est un halogène ou dérivé d'acide sulfonique.

**14.** Composé de la formule (I) ou un éther, ester ou sel d'addition d'acide de celui-ci, tel que défini dans la revendication 1, pour l'utilisation dans le traitement des tumeurs.

**15.** Composé de la formule (I) ou un éther, ester ou sel d'addition d'acide de celui-ci, tel que défini dans la revendication 1, pour l'utilisation dans la préparation d'un médicament pour le traitement des tumeurs.

**Revendications pour l'Etat contractant suivant : AT**

**1.** Procédé pour préparer un composé de la formule (I):

$ArCH_2NHR^1$ (I)

ou un éther de monométhyle ou de monoéthyle de celui-ci, le composé de la formule (I) y compris lesdits éthers ne contenant pas plus de 29 atomes de carbone au total; un de leurs esters; un de leurs sels;
où Ar est un système cyclique aromatique 6,6,6,5-tétracyclique ayant 15 à 17 atomes de cyclique et contenant un cycle à 5 membres dans lequel il y a un hétéroatome, facultativement substitué par un ou deux substituants, lesdits substituants ne contenant pas plus de 4 atomes de carbone au total quand ils sont pris ensemble et sont les mêmes ou différents et choisis parmi halogène; cyano; $C_{1-4}$-alkyle ou $C_{1-4}$-alkoxy, chacun facultativement substitué par hydroxyle ou $C_{1-2}$-alkoxy; $C_{1-2}$-alkyle ou $C_{1-2}$-alkoxy substitué par halogène; un groupe $S(O)_nR^2$ où n est un entier 0,1 ou 2 et $R^2$ est $C_{1-2}$-alkyle facultativement substitué par hydroxyle ou $C_{1-2}$-alkoxy; ou Ar est facultativement substitué par un groupe $NR^3R^4$ ne contenant pas plus de 5 atomes de carbone, où $R^3$ et $R^4$ sont les mêmes ou différents et chacun est un groupe $C_{1-3}$-alkyle ou $NR_3R^4$ forme un anneau hétérocyclique à 5 ou 6 membres, contenant facultativement 1 ou 2 hétéroatomes additionnels;
$R^1$ est

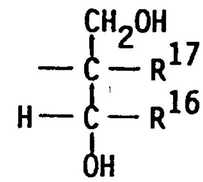

où $R^{16}$ est hydrogène ou méthyle et $R^{17}$ est hydrogène, méthyle ou éthyle, lequel procédé comprend:

(a)- la réduction d'un composé Ar-CH = $NR^1$ ou d'un dérivé protégé de celui-ci, suivie de la déprotection lorsqu'il y a lieu;

(b)- la réduction d'un composé Ar.CO.$NHR^1$, où les groupes hydroxyle sont facultativement protégés, suivie de la déprotection des groupes hydroxyle lorsqu'il y a lieu;

(c)- la réaction d'un composé $ArCH_2L$, où L est un groupe partant, avec un composé $NH_2R^1$;

(d)- tout procédé connu dans le domaine de préparation de composés analogues.

**2.** procédé selon la revendication 1 pour la préparation d'un composé où le système aromatique est choisi parmi le groupe comprenant:

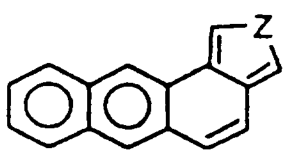

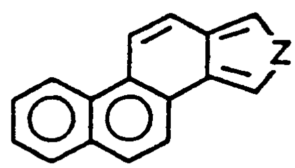

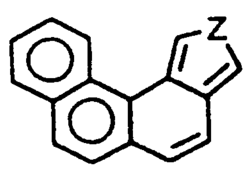

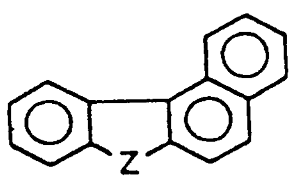

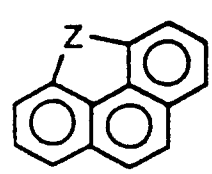

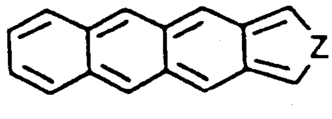

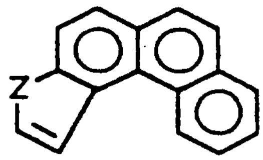

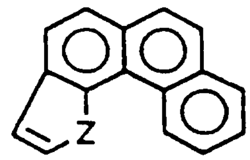

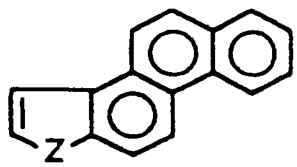

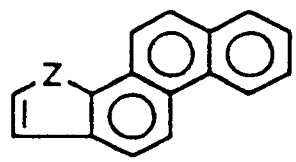

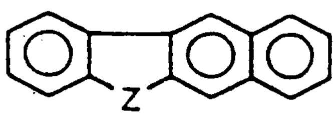

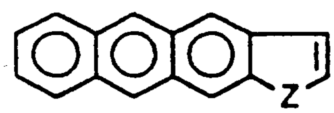

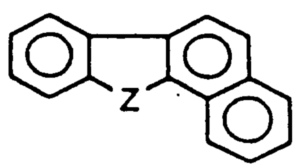

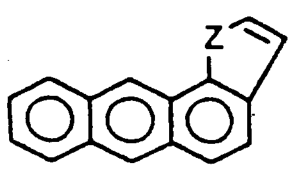

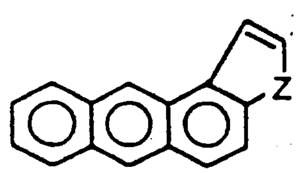

où Z est un hétéroatome.

3. Procédé selon la revendication 1 pour préparer un composé de la formule (I) où Ar est choisi parmi:

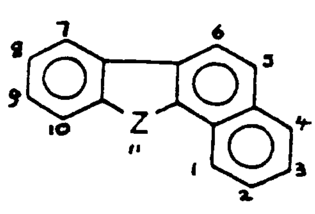

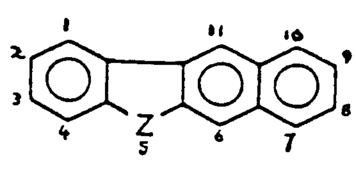

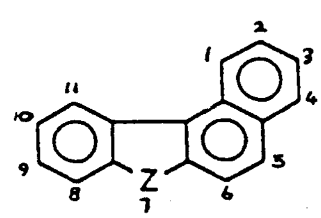

4. Procédé selon la revendication 3 pour préparer un composé où la chaîne latérale $CH_2NHR^1$ est attachée à l'une des positions suivantes:

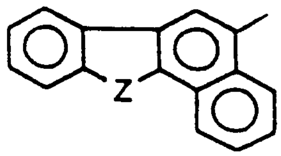
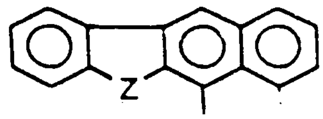
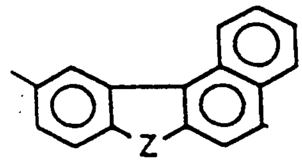

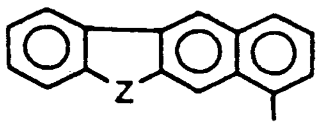
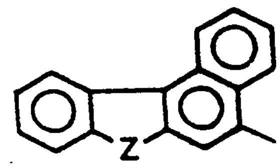

5. Procédé selon la revendication 1 pour préparer un composé où Ar est choisi parmi le groupe comprenant:

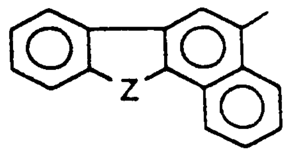

où Z = O, S, NMe ou NEt

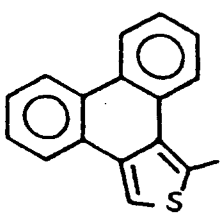
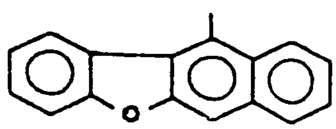
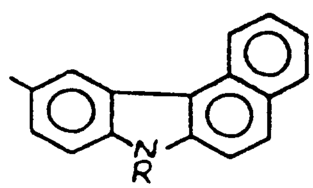

où R = Me ou Et

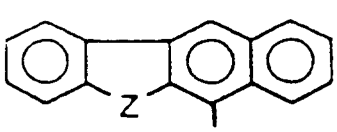
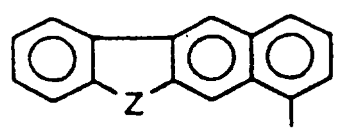

où Z = S ou O, et

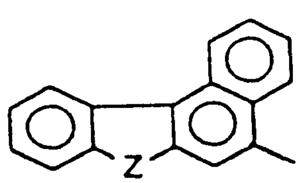

où Z = S, O, NMe, ou NEt

6. Procédé selon la revendication 1 pour préparer un composé de la formule (I) où Ar est:

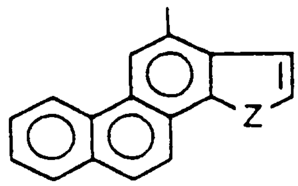

où Z = S, O, NMe, ou NEt

7. Procédé selon la revendication 1 pour préparer un composé de la formule (I) où Ar est le 7H-benzo(c)-carbazol-10-yle ou le benzo(b)naphto(2,1-d)furann-5-yle.

8. Procédé selon la revendication 1 pour préparer un composé choisi parmi:
le 2-[(9-benzo[b]naphto[2,1-d]thiophèn-5-ylméthyl)amino]-2-1,3-propanediol
le 2-[(benzo[b]naphto[2,3-d]furann-6-ylméthyl)amino]-2-méthyl-1,3-propanediol
le 2-[(benzo[b]naphto[1,2-d]furann-5-ylméthyl)amino]-2-méthyl-1,3-propanediol
le 2-[(benzo[b]naphto[2,1-d]furann-5-ylméthyl)amino]-2-méthyl-1,3-propanediol
le 2-méthyl-2-[((7-méthyl-7H-benzo[c]carbazol-10-yl)méthyl)amino]-1,3-propanediol
le 2-((benzo[b]naphto[2,3-d]thiophèn-6-ylméthyl)amino)-2-méthyl-1,3-propanediol
le 2-(5-éthyl-(benzo[b]carbazol-7-ylméthyl)amino)-2-méthyl-1,3-propanediol
le 2-((benzo[b]naphto[2,3-d]furann-11-ylméthyl)amino)-2-méthyl-1,3-propanediol
le 2-((benzo[b]naphto[2,3-d]thiophèn-8-ylméthyl)amino)-2-méthyl-1,3-propanediol
le 2-((5-éthyl)-benzo[b]carbazol-6-ylméthyl)amino)-2-méthyl-1,3-propanediol
le 2-((benzo[b]naphto[2,3-d]thiophèn-7-ylméthyl)amino)-2-méthyl-1,3-propanediol
le 2-((phénanthro[9,10-c]thiophèn-1-ylméthyl)amino)-2-méthyl-1,3-propanediol
le 2-méthyl-2-[(phénanthro[9,10-b]furann-2-ylméthyl)amino)-1,3-propanediol
le 2-[(benzo[b]naphto[1,2-d]thiophèn-5-ylméthyl)amino]-2-méthyl-1,3-propanediol
le 2-méthyl-2-[(phénanthro[4,3-b]furann-2-ylméthyl)amino]-1,3-propanediol
le 2-[(phénanthro[1,2-b]thiophèn-2-ylméthyl)amino]-2-méthyl-1,3-propanediol
le 2-méthyl-2-[(phénanthro[1,2-b]furann-2-ylméthyl)amino]-1,3-propanediol
le 2-méthyl-2-[(phénanthro[4,3-b]furann-7-ylméthyl)amino]-1,3-propanediol
le 2-[(phénanthro[1,2-b]thiophèn-4-ylméthyl)amino]-2-méthyl-1,3-propanediol
le 2-[(phénanthro[1,2-b]furann-4-ylméthyl)amino]-2-méthyl-1,3-propanediol
le 2-[(benzo[b]naphto[2,3-d]furann-7-ylméthyl)amino]-2-méthyl-1,3-propanediol
et leurs éthers de monométhyle ou de monoéthyle, esters et sels d'addition d'acides.

9. Nouveaux intermédiaires chimiques de la formule $ArCH=NR^1$ où Ar et $R^1$ sont tels que définis dans la revendication 1.

10. Nouveaux intermédiaires chimiques de la formule $Ar.CO.NHR^1$ où Ar et $R^1$ sont tels que définis dans la revendication 1.

11. Nouveaux intermédiaires chimiques de la formule $ArCH_2L$ où Ar est tel que défini dans la revendication 1 et L est un halogène ou dérivé d'acide sulfonique.

12. Composition pharmaceutique comprenant un composé de la formule (I), ou un éther, ester ou sel d'addition d'acide de celui-ci, tel que défini dans la revendication 1, et un excipient pharmaceutiquement acceptable.